Europäisches Patentamt

European Patent Office

Office européen des brevets

(19)

(11) **EP 0 307 592 B1**

(12) **EUROPÄISCHE PATENTSCHRIFT**

(45) Veröffentlichungstag und Bekanntmachung des Hinweises auf die Patenterteilung:
**18.11.1998 Patentblatt 1998/47**

(21) Anmeldenummer: **88112130.5**

(22) Anmeldetag: **27.07.1988**

(51) Int Cl.[6]: **C07K 14/10**, C12N 15/00, C12N 1/20, C12P 21/02, A61K 38/55 // (C12N1/20, C12R1:19)

(54) **Varianten des pankreatischen Rinder-Trypsininhibitors, deren Herstellung und Verwendung**

Analogues of the pancreatic bovine trypsin inhibitor, their production and use

Analogues de l'inhibiteur de la trypsine pancréatique bovine, leur production et utilisation

(84) Benannte Vertragsstaaten:
**AT BE CH DE ES FR GB IT LI NL SE**

(30) Priorität: **07.08.1987 GB 8718777**

(43) Veröffentlichungstag der Anmeldung:
**22.03.1989 Patentblatt 1989/12**

(73) Patentinhaber: **BAYER AG**
**51368 Leverkusen (DE)**

(72) Erfinder:
- **Auerswald, Ernst-August, Dr.**
  **D-8000 München 60 (DE)**
- **Bruns, Wolfgang, Dr.**
  **D-5600 Wuppertal 1 (DE)**
- **Hörlein, Dietrich, Dr.**
  **D-5600 Wuppertal 1 (DE)**
- **Reinhardt, Gerd, Dr.**
  **D-5600 Wuppertal 1 (DE)**
- **Schnabel, Eugen, Dr.**
  **D-5600 Wuppertal 1 (DE)**
- **Schröder, Werner, Dr.**
  **D-5600 Wuppertal 1 (DE)**

(56) Entgegenhaltungen:
EP-A- 0 132 732          EP-A- 0 297 362
EP-A- 0 339 942

- BIOCHEMISTRY, Band 16, Nr. 8, 19. April 1977, Seite 1531, The American Chemical Society; N.H. TAN et al.: "Synthesis and characterization of a pancreatic trypsin inhibitor homologue and a model inhibitor"
- THE JOURNAL OF BIOLOGICAL CHEMISTRY, Band 260, Nr. 21, 25. September 1986, Seiten 11451-11455, The American Society of Biological Chemists, Inc., US; E. FIORETTI et al.: "Primary structure and antiproteolytic activity of a Kunitz-type inhibitor from Bovine spleen"
- CHEMICAL ABSTRACTS, Band 104, 1986, Seite 164, Zusammenfassung Nr. 103372t, Columbus, Ohio, US; I.B. KINGSTON et al.: "Sequences encoding two tryspin inhibitors occur in strikingly similar genomic environments", & BIOCHEM. J. 1986, 233(2), 443-50
- EMBO JOURNAL, Band 5, Nr. 12, 1986, Seiten 3219-3225, IRL Press Ltd, Oxford, GB; B. von WILCKEN-BERGMANN et al.: "A synthetic operon containing 14 bovine pancreatic trypsin inhibitor genes is expressed in E. coli"
- CHEMICAL ABSTRACTS, Band 109, 1968, Seite 165, Zusammenfassung Nr. 123459h, Columbus, Ohio, US; E.A. AUERSWALD et al.: "Expression, isolation and characterization of recombinant.....", & BIOL. CHEM. HOPPE-SEYLER 1988, 369(SUPL.), 27-35, & FEBS Saltelite Meeting en Proteinase Inhibitors and Biological Control, Ljubljana/Brdo, Yugoslavia, Juli 4.-7. 1987
- ADV. EXP. MED. BIOL., 1983, 156A (kinins-3,ptA), Seiten 329-337; H. TSCHESCHE et al.: "Peptide/protein inhibitors of tryspin and kallikrein - primary structural requirements"
- JOURNAL OF BIOLOGIGCAL CHEMISTRY, Band 261, Nr. 16, 5. Juni 1986, Seiten 7115-7118, The American Society of Biological Chemists, Inc., US; C. BERMAN MARKS et al.: "Production of native, correctly folded bovine pancreatic trypsin inhibitor by Escherichia coli"

**Beschreibung**

Varianten von pankreatischem Rinder-Trypsininhibitor, hergestellt durch rekombinante DNA-Technologie, Verfahren, Expressionsvektor und rekombinanter Wirt sowie pharmazeutische Verwendung derselben

Einführung und Rahmen der Erfindung

Aprotinin stellt ein gut charakterisiertes basisches Protein mit 58 Aminosäuren dar, das als Inhibitor auf Proteinasen, wie Trypsin, Chymotrypsin, Plasmin und Kallikrein wirkt. Es wurde zu einem wertvollen Arzneimittel, genannt Trasylol [(R)], zur Behandlung verschiedener Erkrankungen, wie z. B. hyperfibrinolytischer Blutung und traumatische Blutung verursachenden Schock.

Es wurde kürzlich gezeigt, daß der Austausch des Lysinrestes in Position 15 des Aprotininmoleküls durch andere Aminosäuren zu wertvollen Proteaseinhibitoren mit einem im Vergleich zu Aprotinin modifizierten Inhibitionsspektrum führt (H. Tschesche et al (1985), Patentanmeldung DE-PS 33 39 693 vom 15.5.85 und auch in Ado. Exp. Med. Bioch. 1983, 156 A, 329-337). Je nach der eingeführten Aminosäure können diese modifizierten Inhibitoren z. B. als Inhibitoren auf Elastasen des Pankreas und der Leukozyten und/oder Plasmakallikrein wirken. Obwohl Aprotininvarianten durch halbsynthetische Umwandlung von Aprotinin erhalten werden können (H. Tschesche et al, DE-OS 33 39 693), sind die dabei erhältlichen Mengen verhältnismäßig gering. Außerdem erlaubt diese Methode nicht einen multiplen Austausch von Aminosäuren zusätzlich zum Lysinrest in Position 15.

Tan und Kauser, Biochemistry, 16(8), 1531-1541 beschreiben Aprotinin varianten mit einem Austausch in der Position 16. Die EP 339 94Z beschreibt Aprotinin varianten mit Austauschen in den Positionen 10-19, 38 41 und 42.

Man hat erkannt, daß die Anwendung rekombinanter DNA und verwandter Technologien den geeignetsten Weg zur Herstellung großer Mengen von Aprotininhomologen mit der gewünschten Spezifität und Inhibitionswirkung darstellen würde.

Der Fachmann auf diesem Gebiet weiß, daß DNA, die für Proteine mit bekannter Aminosäuresequenz kodiert, unter Verwendung der DNA-Sequenz des Genoms oder der cDNA-Sequenz, die komplementär zur mRNA ist, hergestellt werden kann. Ein Austausch von Aminosäuren kann dann e.g. durch ortsspezifische Mutagenese erfolgen.

Eine weitere Möglichkeit, eine DNA, die für ein Protein bekannter Primärstruktur kodiert, zu erhalten, liegt in der Wahl von Codons gemäß dem genetischen Code und der Herstellung eines synthetischen Gens.

Verfahren zur Expression heterologer DNA in einem rekombinanten Mikroorganismus und/oder in eukaryotischen Zellen sind bekannt.

Aufgabe der vorliegenden Erfindung ist es, pharmazeutisch nützliche Polypeptide/Proteine mit einem hohen Spezifitätsgrad zusammen mit einer hohen Inhibitorwirkung zur Verfügung zu stellen. Die genannten Polypeptide stellen Peptide mit der Aminosäuresequenz von Aprotinin und Varianten desselben dar, die durch rekombinante DNA Technologien hergestellt werden können. Die Bezeichnung "Varianten" bezieht sich auf Polypeptide, in welchen eine oder mehrere der Aminosäuren der ursprünglichen Aprotininsequenz durch andere, natürlich vorkommende Aminosäuren ausgetauscht sind.

Folglich betrifft die vorliegende Erfindung Peptide, die die Sequenz von pankreatischem RinderTrypsininhibitor (Aprotinin, BPTI) aufweisen, mit einem Austausch der natürlichen Aminosäure Arg in der Position 17 durch Leu, Ile oder Ala wobei eine oder mehrere der Aminosäuren in den Positionen 15, 16, 18, 34, 39 und 52 durch eine der unten genannten Aminosäure ausgetauscht ist bzw. sind, ausgenommen Val-15-Ser-16-Ile-17-Aprotinin.

Erfindungsgemäß sind Peptide mit den genannt Austauchen in der Position 17, die umfassen:

in Position 15 die Aminosäure Leu, Ile, Val, Phe, Tyr, Trp, Met, Ala, Thr, Ser, Gln, Asn, oder Arg,

in Position 16 die Aminosäure Val, Met, Thr, Ser, Gln, Asn, Gly, oder Arg,

in Position 18 die Aminosäure Leu, Val, Phe, Met, Thr, Glu oder Gly,

in Position 34 die Aminosäure Leu, Ile, Phe, Tyr, Trp, Ala oder Thr,

in Position 39 die Aminosäure Leu, Ile, Val, Phe, Tyr, Trp, Met, Ala, Thr, Ser, Glu, Gln, Asn, Gly, Lys, Asp oder Pro,

und in Position 52 die Aminosäure Leu, Ile, Val, Thr, Ser, Glu, Gln, Asp, Lys oder Arg.

Besonders bevorzugt betrifft die Erfindung

1. Peptide mit den genannten Austauschen in der Position 17, die in Position 15 die Aminosäure Val, Leu oder Ile,

in Position 39 die Aminosäure Glu, Asp, Asn, Thr, Val, Leu, Ile, Gln oder und in
Position 52 die Aminosäure Thr oder Glu aufweisen,

2. Peptide mit den genannten Austauschen in der Position 17, welche in Position 15 die Aminosäure Val, Leu oder Ile,
in Position 39 die Aminosäure Glu und in Position 52 die Aminosäure Thr oder Glu aufweisen,

3. Peptide, mit den genannten Austauschen in der Position 17, welche in
Position 15 die Aminosäure Val, Leu oder Ile,
in Position 39 die Aminosäure Glu und in
Position 52 die Aminosäure Thr oder Glu aufweisen und

4. Peptide mit den genannten Austauschen in der Position 17, welche in
Position 15 die Aminosäure Val oder Leu,
in Position 39 die Aminosäure Glu und in
Position 52 die Aminosäure Thr oder Glu aufweisen.

5. Gemäß der Erfindung sind die folgenden Peptide am meisten bevorzugt:

Val-15-Leu-17-
Val-15 -Leu-17-Glu-52-
Val-15-Leu-17-Thr-52-
Val-15-Leu-17-Glu-39-
Val-15-Leu-17-Glu-39-Glu-52-
Val-15-Leu-17-Glu-39-Thr-52-
Leu-15-Leu-17-
Leu-15-Leu-17-Glu-52-
Leu-15-Leu-17-Thr-52-
Leu-15-Leu-17-Glu-39-
Leu-15-Leu-17-Glu-39-Glu-52-
Leu-15-Leu-17-Glu-39-Thr-52-Aprotinin.

Die Erfindung bezieht sich auch auf Positionen innerhalb des Moleküls, die für bestimmte Expressionsweisen der Proteine über rekombinante DNA-Technologie von Bedeutung sind, insbesondere Position 52.

Die Erfindung betrifft im weiteren Polypeptide/Proteine mit den vorstehend angegebenen Sequenzen, die zusätzlich Methionin in Position -1 und/oder ein Leaderpeptid aufweisen. Die Bezeichnung "Leaderpeptid" betrifft in bezug auf diese Anmeldung nicht nur Signalsequenzen, welche die Sekretion des Expressionsproduktes steigern, sondern auch Sequenzen, die eine Signalsequenz und eine Linkersequenz, welche dem Aprotinin oder den Sequenzen der Aprotininvariante vorausgehen, umfassen. Außerdem bezieht sich die Bezeichnung "Leadersequenz" auf Sequenzen an beiden Enden des Moleküls, wobei diese eine hohe Expression gewährleisten und/oder zur leichteren Reinigung der Moleküle dienen.

Die vorliegende Erfindung betrifft auch Polypeptide/Proteine mit den unter 5 genannten Sequenzen, die jedoch an einem oder an beiden Enden des Moleküls um eine oder mehrere Aminosäuren verkürzt sind, wobei die Inhibitionswirkung teilweise oder vollkommen beibehalten wird.

Homologe (Varianten) von Aprotinin, wie sie vorstehend spezifiziert sind, können therapeutisch bei Erkrankungen eingesetzt werden, die auf dem Vorliegen überschüssiger Mengen an Proteinasen, e. g. pankreatischer Elastase (Pankreatitis), Serumelastase (Arteriosklerose), Leukozytenelastase bei chronischer und akuter Entzündung mit einer Schädigung des Bindegewebes, bei Schäden der Gefäßwände, nekrotischen Erkrankungen und Degenerierung des Lungengewebes, beruhen. Ebenso wichtig ist die Rolle, die dabei lysosomale Enzyme spielen, insbesondere Leukozytenelastase, bei Entzündungsreaktionen aufgrund immunologischer Prozesse, e. g. rheumatoide Arthritis, oder als Myokard-Depressivumfaktor oder bei Schocksyndromen.

Es wurde gefunden, daß die Anwendung von rekombinanter DNA und verwandter Technologien den geeignetsten Weg darstellen, um große Mengen an Aprotininhomologen mit der gewünschten Spezifität und Inhibierungswirkung zu produzieren.

Es wurde auch gezeigt, daß Homologe von Aprotinin durch rekombinante DNA-Technologie unter Verwendung eines Konstruktes erhalten werden können, in dem synthetische Gene an das lac Z Gen gebunden (E. Auerswald et al, (1985), Patentanmeldung GB-8607523) oder nicht-fusioniert sind (v. Wilcken-Bergmann et al (1986), EMBO J., 5, 3219-3225). Außerdem wurde der natürliche Kodierungsbereich von Aprotinin als eine Fusion mit der Signalsequenz des pho A Gens (alkalische Phosphatase) exprimiert (B. Marks et al (1986), J. Biol. Chem., 204, 7115-7118).

Oft ist es einfacher, eine hohe Expression eines bestimmten kleinen heterologen Proteins in einem bakteriellen Wirt zu erreichen, indem man Genfusionen verwendet. Dies ist auf eine Vielzahl von Gründen zurückzuführen. Faktoren, die offensichtlich die Akkumulierung von heterologen Polypeptiden in einer Wirtszelle beeinflussen, schließen folgende ein:

(1) Das heterologe Polypeptid kann durch Proteinasen der Wirtszelle effiziert abgebaut werden.

(2) Das heterologe Polypeptid kann eine toxische Wirkung auf die Zelle haben.

Obwohl eine große Zahl heterologer Proteine mit Molekulargewichten über 10.000 Dalton mit Erfolg in E. coli intrazellulär exprimiert wurde, gelang es bisher nur relativ wenige, kleine heterologe Polypeptide gut zu exprimieren, obwohl man dazu viele Versuche unternommen hat.

In dem Bestreben, dieses Problem zu überwinden, hat man sich häufig der Fusionspolypeptide bedient. Die Nachteile dieser Methode sind unter anderem folgende:

(1) Die insertierte strukturelle Sequenz muß zum AUG Startcodon des Fusionspartners im richtigen Leseraster sein.

(2) Das heterologe Polypeptid muß aus dem Fusionspolypeptid chemisch oder enzymatisch abgespalten werden. Aus diesem Grunde darf das gewünschte Protein keine der jeweiligen Spaltstellen enthalten. Dies stellt in vielen Fällen ein schwerwiegendes Problem dar.

Es ist jedoch manchmal möglich, kleine Polypeptide unter Verwendung von Plasmiden zu exprimieren, die vielzählige Kopien des Gens enthalten (v. Wilcken-Bergman et al (1986), EMBO J., 5, 3219-3225).

Eine weitere Möglichkeit, dieses Problem des Abbaus und/ oder der Toxizität zu überwinden, besteht in der Expression des heterologen Proteins in einem Sekretionssystem. Es können Sekretionssysteme von Bakterien, Hefen oder Pilzen verwendet werden. Um eine Sekretion zu erreichen, muß eine geeignete Leadersequenz an das 5'-Ende des Gens von Aprotinin oder den Aprotininvarianten so angebracht werden, daß die korrespondierende Aminosäuresequenz vor dem N-terminalen Ende eine Spleißstelle für die Signal-Peptidase umfaßt, welche das zu sezernierende Protein spleißt.

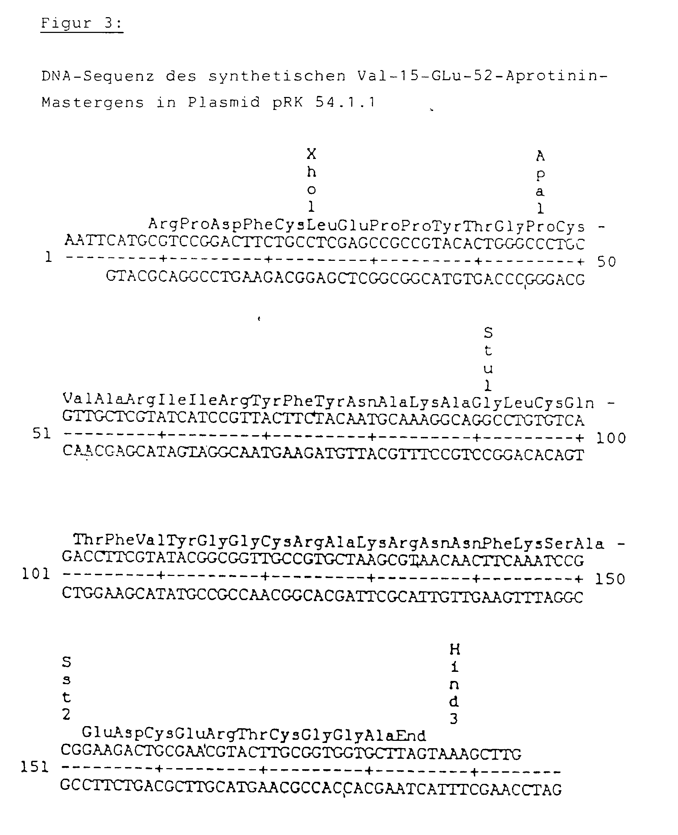

Die vorliegende Erfindung betrifft synthetische DNA-Sequenzen, die für Aprotininhomologe kodieren. Sie betrifft insbesondere eine DNA, die nachfolgend als "Mastergen" bezeichnet wird, und welche die in Fig. 3 gezeigte Sequenz und/oder funktionelle Äquivalente derselben aufweist. Die Bezeichnung "funktionelle Äquivalente" bedeutet in bezug auf die Anmeldung auch Derivate der vorstehenden DNA-Sequenz, in denen in einigen Codons eine, zwei oder drei der Basen durch eine andere Base ausgetauscht sind, ohne daß dies eine Wirkung auf die in das Protein einzubauende Aminosäure hat (Entartung des genetischen Codes).

Um Varianten des Aprotininmoleküls herzustellen, wird das Mastergen mit Hilfe von rekombinanten DNA-Technologien (e. g. ortsspezifische Mutagenese) in einer Weise modifiziert, daß das Codon für eine bestimmte Aminosäure durch das Codon für eine andere Aminosäure ausgetauscht wird. Mit Hilfe dieser Methode können die verschiedenen DNA-Sequenzen, die für die Aprotininhomologe kodieren (s. die o.g. Aprotinin-Varianten) gemäß der Erfindung erhalten werden.

Die Codons für einen solchen Austausch sind die Codons für die bevorzugten Aminosäuren, wie sie auf den Seiten 3, 4, 5 und 6 aufgelistet sind. Je nach dem verwendeten Expressionssystem kann die DNA der vorliegenden Erfindung auch eine DNA sein, die für eine der Polypeptidvarianten, wie sie in Tabelle 1 aufgeführt sind, kodiert, wobei diese stromaufwärts am 5'-Ende weitere Sequenzen aufweist. Eine weitere Aufgabe der vorliegenden Erfindung stellen Expressionsvektoren (Plasmide) dar, welche die DNA, die für die Polypeptide kodiert, enthalten. Diese Plasmide dienen zur Transformation eines Wirtsorganismus. Die vorliegende Erfindung betrifft auch derart transformierte Organismen. Dem Fachmann sind eine große Zahl von Organismen, die zur Transformation geeignet sind, bekannt. Die Art des Plasmids ist in der Hauptsache von dem zu verwendenden Wirtsorganismus abhängig.

Der Wirtsorganismus, der mit dem Plasmid, welches die DNA-Sequenzen der vorliegenden Erfindung enthält, transformiert ist, dient zur Herstellung der Aprotininhomologen. Die Herstellung umfaßt die folgenden Schritte:

(1) Züchten des Wirtsorganismus unter geeigneten Bedingungen,
(2) Gewinnung der Peptide aus der Kultur und
(3) Reinigen der Peptide.

Die Reinigung der Polypeptide kann nach bekannten Verfahren der Proteinchemie erfolgen, e. g. Ausfällung, Chromatographie und Elektrophorese. Die vorstehend genannten Linkerpeptide können bei solchen Reinigungen hilfreich sein, da die Charakteristiken des Linkers dazu verwendet werden können, die Reinigung zu erleichtern (ein Beispiel wird von S.J. Bruver und H.M. Sassenfeld angegeben (1985), Trends in Biotechnology, 3, 119-122).

Die vorliegende Erfindung betrifft auch pharmazeutische Zubereitungen und Präparationen, welche die vorstehend aufgeführten Peptide umfassen, sowie die Verwendung dieser Peptide bei der Herstellung pharmazeutischer Zubereitungen. Diese pharmazeutischen Zubereitungen sind für die vorstehend beschriebenen Indikationen besonders geeignet.

Die pharmazeutischen Präparationen enthalten außer nichttoxischen, inerten, pharmazeutisch geeigneten Hilfsstoffen eine oder mehrere Verbindungen gemäß der Erfindung; die Erfindung umfaßt außerdem Verfahren zur Herstellung dieser Präparationen.

Die vorliegende Erfindung umfaßt auch pharmazeutische Präparationen in Dosiseinheiten. Dies bedeutet, daß die Präparationen in Form einzelner Teile vorliegen, z. B. als Tabletten, beschichtete Tabletten, Kapseln, Pillen, Suppositorien und Ampullen, deren Gehalt an Wirkstoff einem Bruchteil oder einem Vielfachen einer Einzeldosis entspricht. Die Dosiseinheiten können z. B. eine, zwei, drei oder vier einzelne Dosen oder eine halbe, eine drittel oder eine viertel Dosis oder eine Einzeldosis enthalten. Eine Einzeldosis enthält bevorzugt die Menge an Wirkstoff, die bei einer Verabreichung gegeben wird, und die üblicherweise einer ganzen, der Hälfte, einem Drittel oder einem Viertel einer täglichen Dosis entspricht.

Unter nicht-toxischen, inerten, pharmazeutisch geeigneten Hilfsstoffen versteht man feste, halbfeste oder flüssige Verdünnungsmittel, Füllstoffe und Formulierungshilfsmittel aller Art.

Als bevorzugte pharmazeutische Präparationen sind zu nennen: Tabletten, beschichtete Tabletten, Kapseln, Pillen, Granulate, Suppositorien, Lösungen, Suspensionen und Emulsionen, Pasten, Salben, Gele, Cremen, Lotionen, Pulver und Sprays.

Tabletten, beschichtete Tabletten, Kapseln, Pillen und Granulate können den Wirkstoff oder die Wirkstoffe zusammen mit gebräuchlichen Hilfsstoffen enthalten, wie z. B. (a) Füllstoffe und Streckmittel, wie Stärken, Lactose, Sucrose, Glucose, Mannit und Siliziumdioxid, (b) Bindemittel, wie Carboxymethylcellulose, Alginate, Gelatine und Polyvinylpyrrolidon, (c) anfeuchtende Mittel, wie Glycerin, (d) Sprengmittel, wie Agar-Agar, Kalziumcarbonat und Natriumcarbonat, (e) Lösungsverzögerer, Paraffin und (f) Adsorptionsbeschleuniger, wie quaternäre Ammoniumverbindungen, (g) Netzmittel, wie Cetylalkohol und Glycerinmonostearat, (h) Adsorbenzien, wie Kaolin und Bentonit und (i) Gleitmittel, wie Talk, Kalzium- und Magnesiumstearat und feste Polyethylenglykole, oder Gemische der Substanzen wie sie unter (a) bis (i) aufgeführt sind.

Die Tabletten, beschichteten Tabletten, Kapseln, Pillen und Granulate können mit üblichen Überzügen und Umhüllungen versehen sein, welche gegebenenfalls Opakisierungsmittel enthalten; sie können auch in solchen Zubereitungen vorliegen, die den Wirkstoff oder die Wirkstoffe vorzugsweise nur in einem bestimmten Teil des Verdauungstraktes, gegebenenfalls in verzögerter Weise, freisetzen, wobei als Beispiele für geeignete Materialien zum Einbetten Polymersubstanzen und Wachse genannt werden.

Der Wirkstoff oder die Wirkstoffe, gegebenenfalls zusammen mit einem oder mehreren der vorstehend genannten Trägerstoffe kann bzw. können auch in Form von Mikrokapseln vorliegen.

Suppositorien können neben dem Wirkstoff oder den Wirkstoffen die üblichen wasserlöslichen oder wasserunlöslciehn Trägerstoffe enthalten, z. B. Polyethylenglykole, Fette, z. B. Kakaofett, und höhere Ester (z. B. $C_{14}$-Alkohol mit $C_{16}$-Fettsäure) oder Gemische dieser Substanzen.

Salben, Pasten, Cremen und Gele können übliche Exzipienten neben dem Wirkstoff bzw. den Wirkstoffen enthalten, z. B. tierische oder pflanzliche Fette, Wachse, Paraffine, Stärke, Traganth, Cellulosederivate, Polyethylenglykole, Silikone, Bentonite, Siliziumdioxid, Talk und Zinkoxid, oder Gemische dieser Substanzen.

Pulver und Sprays können übliche Trägerstoffe außer dem Wirkstoff oder den Wirkstoffen enthalten, z. B. Lactose, Talk, Siliziumdioxid, Aluminiumhydroxid, Kalziumsilikat und Polyamidpulver, oder Gemische dieser Substanzen. Die Sprays können außerdem übliche Treibmittel enthalten, z. B. Chlorfluorkohlenwasserstoffe.

Lösungen und Emulsionen können übliche Trägerstoffe neben dem Wirkstoff oder den Wirkstoffen enthalten, wie Lösungsmittel, solubilisierende Agenzien und emulgierende Agenzien, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsamenöl, Erdnußöl, Maiskeimöl, Olivenöl, Rizinusöl und Sesamöl, Glycerinformal, Tetrahydrofurfurylalkohol, Polyethylenglykole und Fettsäureester von Sorbitan, oder Gemische dieser Substanzen.

Für die parenterale Verabreichung können die Lösungen und Emulsionen auch in steriler, mit dem Blut isotoner Form vorliegen.

Suspensionen können übliche Trägerstoffe neben dem Wirkstoff oder den Wirkstoffen enthalten, wie flüssige Verdünner, z. B. Wasser, Ethylalkohol oder Propylenglykol, suspendierende Agenzien, z. B. ethoxylierte Isostearylalkohole, Polyoxyethylen-sorbit -sorbit und Sorbitanester, mikrokristalline Cellulose, Aluminiummetahydroxid, Bentonit, Agar-Agar und Traganth, oder Gemische dieser Substanzen.

Die genannten Formulierungen können auch Farbstoffe, Konservierungsmittel und Additive enthalten, die den Geruch und Geschmack verbessern, z. B. Pfefferminzöl und Eukalyptusöl, sowie Süßungsmittel, wie Saccharin.

Die therapeutisch wirksamen Verbindungen sollen vorzugsweise in den vorstehend genannten pharmazeutischen Präparationen in einer Konzentration von ca. 0,1 bis 99,5 Gew.-%, vorzugsweise von ca. 0,5 bis 95 Gew.-%, bezogen auf das Gesamtgewicht, vorliegen.

Die vorstehend genannten pharmazeutischen Präparationen können auch andere pharmazeutische Wirkstoffe neben den Verbindungen gemäß der Erfindung enthalten.

Die vorstehend genannten pharmazeutischen Präparationen werden in üblicher Weise nach bekannten Verfahren hergestellt, z. B. durch Vermischen des Wirkstoffes oder der Wirkstoffe mit dem oder den Trägerstoff(en)

Die Wirkstoffe oder die pharmazeutischen Präparationen können lokal, oral, parenteral, intraperitoneal und/oder rektal verabreicht werden, vorzugsweise oral oder parenteral, wie intravenös oder intramuskulär.

Im allgemeinen hat es sich sowohl in der Human- als auch in der Veterinärmedizin als vorteilhaft erwiesen, den Wirkstoff oder die Wirkstoffe gemäß der Erfindung in Gesamtmengen von ca. 0,5 bis ca. 500, vorzugsweise von 5 bis 100 mg/kg Körpergewicht alle 24 h zu verabreichen, gegebenenfalls in Form von mehreren Einzelverabreichungen, um die gewünschten Ergebnisse zu erzielen. Eine Einzelverabreichung enthält den Wirkstoff oder die Wirkstoffe gemäß der Erfindung vorzugsweise in Mengen von ca. 1 bis 250, insbesondere von 3 bis 60 mg/kg Körpergewicht. Es kann jedoch erforderlich sein, von den genannten Dosen abzuweichen, und zwar insbesondere in Abhängigkeit von der Natur und dem Körpergewicht des zu behandelnden Individuums, der Natur und der Schwere der Erkrankung, der Art der Präparation und der Verabreichungsweise der Medizin sowie der Zeit oder der Zeitdauer, über die die Verabreichung stattfindet.

Somit kann es in manchen Fällen genügen, weniger als die vorstehend genannte Menge an Wirkstoff zu verabreichen, während in anderen Fällen die vorstehend genannte Menge an Wirkstoff überschritten werden muß. Die speziell erforderliche optimale Dosis und der Verabreichungstyp der Wirkstoffe können vom Fachmann aufgrund seines Fachwissens ohne weiteres bestimmt werden.

Synthetische Gene

Im folgenden wird die Strategie zur Synthese von Genen, die für Aprotinin und Aprotininhomologe kodieren, beschrieben:

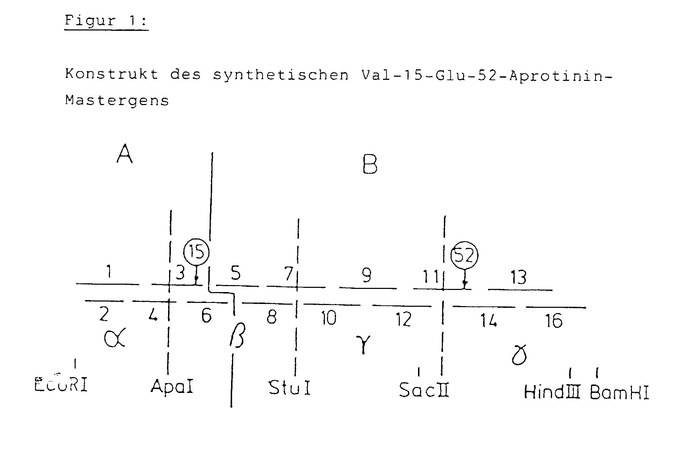

Die bekannte Proteinsequenz von Aprotinin und dessen Homologen und der genetische Code dienten dazu, die DNA-Sequenz, die für diese Polypeptide kodiert, festzulegen. Sämtliche mögliche Basensubstitutionen (Entartung des genetischen Codes) wie auch alle potentiellen Restriktionsstellen wurden für die Konstruktion der synthetischen DNA-Sequenz berücksichtigt. Die sich dabei ergebende, prinzipielle Konstruktion für ein synthetisches Aprotiningen und dessen Homologe wird in Fig. 1, 2 und 3 gezeigt. Das synthetische Mastergen besteht aus vier Blökken (mit alpha, beta, gamma, delta bezeichnet), wobei jeder Erkennungsstellen für Restriktionsendonukleasen an beiden Enden (s. auch Beispiel 3, Fig. 1) aufweist. Dies erlaubt eine einfache Modifizierung und Änderung der DNA-Sequenzen (Codonbenutzung, Mutationen, Amplifizierung von Genen, Proteinbautechnik) für z. B. nichtfusionierte Expression oder Expression als Fusionsproteine. Fusionsproteine werden so konstruiert, daß das gewünschte Protein aus der Fusion durch enzymatische oder chemische Methoden freigesetzt werden kann oder während der Sekretion in den periplasmatischen Raum freigesetzt wird.

Das Gesamtspektrum der Proteinbautechnik wird mit dieser Konstruktion möglich. Eine Amplifizierung des Gens kann durch Zufügen geeigneter Linkersequenzen erreicht werden. Ein Austausch von Aminosäuren ist in sämtlichen Positionen möglich, ausgenommen denjenigen, die Teil einer Erkennungstelle für eine Restriktionsendonuklease sind. Diese (und alle anderen) können durch den Fachmann z. B. durch ortsspezifische Mutagenese verändert werden. Das für die Klonierung der synthetischen Gene ausgewählte Plasmid war pUC 8 (J. Vieira und J. Messing (1982), Gene, 19, 259). Das Plasmid ist im Handel von P-L Biochemicals erhältlich. Die synthetischen Gene wurden in pUC 8 kloniert und direkt mit Hilfe der Super-coil-DNA-Sequenzierungsmethode sequenziert (Literaturstelle von K.J. Chen und P.H. Seeburg (1985), DNA, 4, 165-170).

Expressionsplasmide

Zur Expression von Aprotininhomologen als Fusionsproteine wurden Plasmide verwendet, in welchen das entsprechende Gen an (1) das 3'-Ende des Kodierungsbereiches des lac Z Gens (beta-Galactosidase) (U. Rüther und B. Müller-Hill (1983), EMBO J., 2, 1791-1794) oder an (2) den Codon der Aminosäure 98 der Polymerase des Phagen MS-2 (E. Remault et al (1981), Gene, 15, 81-93) gebunden war.

Zur Expression von Aprotininhomologen, welche in den periplasmatischen Raum oder in das Medium sezerniert wurden, wurde das jeweilige Gen in einen alpha-Amylase-Sekretionsvektor kloniert (M.A. Sullivan et al (1984), Gene, 29, 21-26). Das Gen wurde durch Einführung einer XbaI-Spaltstelle am 5'-Ende des Blockes alpha modifiziert. Eine derartige Konstruktion ermöglicht die Abspaltung des primären Translationsproduktes während der Sekretion durch die endogene Leaderpeptidase unter Bildung des gewünschten Aprotinins oder der Aprotininvariante.

Die Insertion der synthetischen Gene von Aprotinin und Aprotininvarianten in die richtigen Klonierungsstellen und im korrekten Leseraster führt in allen Fällen zu den gewünschten Fusionsproteinen.

E. coli Stamm RRI Δ M15 wurde mit jeweils einem der folgenden drei Expressionsplasmide pES 44.1.1, pES 45.1.3 oder pCH 2742 transformiert und am 26. Juni 1987 in der Deutschen Sammlung von Mikroorganismen, D-3400 Göttingen, Grisebachstr. 8 / BRD unter den angegebenen Hinterlegungsnummer hinterlegt:

E. coli RRI ΔM15 pES 44.1.1 DSM 4157
E. coli RRI ΔM15 pES 45.1.3 DSM 4158
E. coli RRI ΔM15 pCH 2742 DSM 4159

Wirksamkeit der Aprotininvarianten

Durch Änderung der Aminosäuresequenz von Aprotinin in den in Tabelle 1 angegebenen Positionen konnte überraschenderweise gefunden werden, daß damit nicht nur die Inhibitionswirkung gegenüber humaner Leukozytenelastase in signifikanter Weise erhöht werden konnte, sondern es wurden auch Varianten gefunden, die potente Inhibitoren anderer Serinproteasen, wie humanes Cathepsin G oder humane pankreatische Elastase I, darstellen.

Beispiele von Aprotininvarianten, die eine erhöhte Inhibitionswirkung gegenüber humaner Leukozytenelastase zeigen, sind solche, die in Position 17 die Aminosäure Leucin aufweisen. Diese ergaben einen $K_i$-Wert, der um fast eine Größenordnung besser ist als bei Varianten, die in Position 17 die ursprüngliche Aminosäure Arginin aufweisen. Die Wirksamkeitsverbesserung dieser Inhibitoren wurde auch an relevanteren Testmodellen demonstriert, e. g. in einem Abbauassay unter Verwendung von subendothelialer Matrix oder in einem Modell akuter pulmonärer Entzündung bei Hamstern.

Beispiele von Aprotininvarianten, die zusätzlich zu humaner Leukozytenelastase andere wichtige Leukozytenproteasen, wie Cathepsin G, inhibieren, sind Varianten, die in den Positionen 15 und 17 die Aminosäure Leucin aufweisen.

Beispiele von Aprotininvarianten, die humane pankreatische Elastase I inhibieren, sind solche, die in Position 39 die Aminosäure Glutamat und in Position 17 Leucin aufweisen.

Materialien und Methoden

Die synthetischen Gene, rekombinanten Plasmide und Expressionsvektoren, welche die synthetischen Gene enthalten, können mit Hilfe folgender Materialien und Methoden hergestellt und charakterisiert werden:

Materialien

1) Enzyme
DNA-Polymerase,(Klenow); T4-DNA-Ligase (0,9 Einheiten/ µl); Lysozym; RNase A und Polynukleotidkinase von Boehringer, Mannheim.
Restriktionsenzyme von Boehringer, Mannheim, Bethesda Research Labs und Biolabs wurden entsprechend den Instruktionen der Hersteller verwendet.

2) Reagenzien
ATP, dATP, TTP von Sigma; DTE, Thymidin von Serva, Heidelberg; Saccharose von Bethesda Research Labs; Diaminopimelinsäure, Rubidiumchlorid von Sigma; alle anderen Reagenzien waren pro analysi (Merck, Darmstadt und/oder Sigma).

3) DNA/Plasmide
Plasmid pUC 8; 5'-phosphorylierter Bam H1 Linker von P-L Biochemicals (Pharmacia);
Plasmid pUR 278 (U. Rüther und B. Müller-Hill (1983), EMBO J., 2, 1791-1974);
Plasmid pPLc24, erhalten von W. Fiers, Universität Gent, Belgien (Konstrukt von pPLc24: Remault et al, Gene 15, 81-93 (1981).

4) Stämme
E. Coli RR1 Δ M15 von ATCC (Nr. 35 102)
E. coli C 600[pcl 857], erhalten von W. Fiers, Universität Gent, Belgien (Konstrukt von pcl 857: Remault et al, Gene 22, 103-113 (1983); E. coli C600: ATCC 23724).
Bacillus subtilis von DSM (Nr. 704).

5) <u>Medien</u>

Bacto-Trypton; Bacto-Hefe-Extrakt; Bacto-Agar von DIFCO

LB-Medium (für 1 1): 10 g Bacto-Trypton, 5 g Bacto-Hefe-Extrakt, 10 g NaCl, eingestellt mit NaOH auf pH 7,5.

kappa 1776-Medium (für 1 1): 25 g Bacto-Trypton, 7,5 g Bacto-Hefe-Extrakt, 20 ml 1 M tris-HCl pH 7,5, aufgelöst in 950 ml destilliertem Wasser und im Autoklaven behandelt.

Nach dem Abkühlen wurden zugegeben: 5 ml 1M Magnesiumchlorid, 10 ml 1 %ige Diaminopimelinsäure, 10 ml 0,4 %iges Thymidin, 25 ml 20 %ige Glukose (sämtliche der zugegebenen Lösungen wurden durch Filtration sterilisiert).

Agar-Platten wurden hergestellt durch Zugabe von 15 g Bacto-Agar zu 1 1 des geeigneten Mediums.

6) <u>Antibiotika</u>

Chloramphenicol und Kanamycin-sulfat von Boehringer, Mannheim.

Ampicillin und Tetracyclin von Serva, Heidelberg.

7) <u>Puffer und Lösungen</u>

| 10 mM ATP | in Wasser |
|---|---|
| 10X-Ligase-Mix: | 0,5 M Tris-HCl (pH 7,4); 0,1 M MgCl$_2$; 0,1 M DTE; 10 mM ATP |
| 10X SP-50: | 100 mM Tris=HCl (pH 7,5); 100 mM MgCl$_2$; 500 mM NaCl; 10 mM DTT |
| 10X SP-100: | 100 mM Tris-HCl (pH 7,5); 100 mM MgCl$_2$; 1 M NaCl; 10 mM DTT |
| 10X SP-O: | 100 mM Tris-HCl (pH 7,5); 100 mM MgCl$_2$; 10 mM DTT |
| 20X E-Puffer: | 0,8 M Tris; 0,4 M Natriumacetat; 40 mM EDTA; pH 8,3 |

Transformationspuffer (wie folgt hergestellt): 15 g Saccharose, 1 ml 3,5 M KOH, 1 ml 1 M CaCl$_2$, 2 ml 5,0 M RbCl, werden mit Aqua bidest. auf 50 ml gebracht, mit 10 %iger Essigsäure auf pH 6,2 eingestellt,

es wird 1ml 4,5 M MnCl$_2$ zugegeben, mit 10 %iger Essigsäure auf pH 5,8 eingestellt, mit Aqua bidest. auf 100 ml gebracht und steril filtriert.

| TE-Puffer: | 10 mM Tris-HCl pH 8,0, 0,1 mM EDTA |
|---|---|
| 10X NT-Puffer: | 0,5 M Tris-HCl pH 7,2; 0,17 MgSO$_4$, 1 mM DTE |
| Lysozym-Mix: | 50 mM Glucose, 2 mg/ml in 1 mM EDTA, 10 mM Tris-HCl pH 8,0, vor Anwendung frisch hergestellt |
| Phenol/Sevag: | Gemisch aus 1 Volumen 80 %igen Phenol und 1 Volumen Sevag (Chloroform: iso-Amylalkohol, 24:1) |
| TEABC-Puffer: | 1 M Triethylamin in destilliertem Wasser, pH mit gasförmigem CO$_2$ eingestellt auf 7,5 |
| 10X PNK-Mix: | 0,5 Tris-HCl (pH 7,6), 0,1 M MgCl$_2$) 50 mM DTE, 1 mM EDTA |
| 10X Ligase-Mix: | 0,5 M Tris-HCl (pH 7,4), 0,1 M MgCl$_2$, 0,1 M DTE, 10 mM ATP |

<u>Methoden</u>

Es wurde nach Standardmethoden der rekombinanten DNA-Technik gearbeitet, wie sie in Maniatis et al (1982), Molecular Cloning Cold Spring Harbor Laboratory, Cold Spring Harbor, USA beschrieben werden:

<u>Standard-Ethanolfällung</u>

DNA-Pellets wurden aufgelöst oder Lösungen wurden auf 0,3 M Natriumacetat eingestellt; es wurden 2 Volumen Ethanol zugegeben; 15 min bei -70°C inkubiert und zentrifugiert. Die Pellets wurden zweimal mit 80 %igem Ethanol gewaschen und im Vakuum getrocknet.

Standard-Phenolextraktion

Die Lösungen wurden gründlich mit Phenol/Sevag (1:1) gemischt, zentrifugiert und die Phenolphase mit 1/10 Volumen an TE-Puffer oder Wasser reextrahiert. Die wäßrigen Phasen wurden vereinigt.

Standard-Dephosphorylierung von DNA

Vollkommen abgebaute und gereinigte DNA wurde in Wasser aufgelöst und auf 1X CIP-Puffer eingestellt (Standard-Gesamtvolumen 48 µl). Die Reaktion wurde bei 37°C durch Zugabe von 1 µl (20 Einheiten) Phosphatase aus Kälberdarm (CIP) gestartet; nach 30 min wurde erneut 1 µl CIP zugegeben. Die Reaktion wurde nach 1 h durch Zugabe von 5 µl 50 mM EGTA gestoppt, und es wurde 10 min bei 65°C inkubiert. Zur Dephosphorylierung von DNA mit glatten Enden oder versetzten 5'-Enden wurden wiederholt Inkubationen jeweils 15 min bei 37°C und 15 min bei 56°C durchgeführt. Die DNA wurde mit Phenol/Sevag extrahiert und mit Ethanol ausgefällt.

Standard-Ligierung

Für Standard-Ligierungen wurde ein fünffacher molarer Überschuß des Fragmentes gegenüber dem Vektor verwendet. Die Endkonzentration der DNA betrug 25 µg/ml. Die DNA wurde in einer kleinen Menge TE-Puffer aufgelöst. Die Ligierung erfolgte mit T4-DNA-Ligase in 1X Ligase-Mix (50 mM Tris-HCl pH 7,4, 10 mM $MgCl_2$, 10 mM DTE, 1 mM ATP) in einem Standard-Volumen von 30 µl 16 h bei 14°C.

Standard-Spaltung mit Restriktionsendonuklease

Die Spaltungen mit Restriktionsendonukleasen wurden in der Hauptsache entsprechend den Angaben der Hersteller durchgeführt.

Gereinigte, salzfreie DNA wurde in Puffer (SP-O, SP-50 oder SP-100, je nach dem verwendeten Enzym) aufgelöst und mit einer geeigneten Enzymmenge gespalten. Das Material wurde dann mit Phenol extrahiert und mit Ethanol ausgefällt.

Standard-Isolierung von DNA-Fragmenten nach Agarosegel-Elektrophorese

Die DNA-Fragmente wurden durch Agarosegel-Elektrophorese aufgetrennt (s. T. Maniatis et al, 1982, Cold Spring Harbor Laboratory, Molecular Cloning), mit Ethidiumbromid angefärbt und unter langwelligem UV-Licht herausgeschnitten. Die herausgeschnittenen Teile wurde in einen Dialyseschlauch gegeben, mit 0,5X E-Puffer aufgefüllt (Volumenverhältnis Puffer:Gelschnitte 1,5:1); die Gelschnitten müssen gut von Puffer umgeben sein. Der verschlossene, luftblasen-freie Schlauch wurde in eine mit 0,5X E-Puffer gefüllte Elektrophoresekammer gegeben. Die Elektrophorese wurde 30 min bei 200 V durchgeführt, dann wurde der Strom 30 s lang umgepolt, um die DNA von der Wand des Dialyseschlauches freizusetzen. Der die Gelschnitte umgebende Puffer wurde sorgfältig entfernt und die DNA daraus auf DEAE-Cellulose oder DE 52 Säulen (s. oben) gereinigt.

Standard-Transformationsverfahren

Transformationen wurden entsprechend dem Verfahren von D. Hanahan (1983), J. Mol. Biol., 166, 557-580) durchgeführt.

1 ml einer 20 ml Übernacht-Kultur des Wirtstammes, inokuliert mit einer einzigen Kolonie und in kappa 1776 Medium gezüchtet (37°C, Schüttler mit 200 Upm) wurde zum Inokulieren von 100 ml vorgewärmtem (37°C) kappa 1776 Medium verwendet.

Diese Kultur wurde unter den gleichen Bedingungen gezüchtet. Das Zellwachstum wurde bei 0,2 OD (500 nm) gestoppt. Nach Abkühlen auf 4°C und Zentrifugieren wurde das Zellpellet in 20 ml eiskaltem Transformationspuffer resuspendiert und 5 min bei 0°C inkubiert. Die Suspension wurde erneut zentrifugiert (3000 Upm, 4°C, 15 min) und das Pellet in 4 ml eiskaltem Transformationspuffer resuspendiert. Nach Zugabe von 7 µl DMSO zu 200 ml aliquote Teile wurden die Zellen 15 bis 60 min in Eiswasser inkubiert. Zu einem solchen aliquoten Teil kompletter Zellen wurde DNA, aufgelöst in 20 µl TE-Puffer, zugegeben und das Gemisch 20 min in Eiswasser und dann 3 min bei 42°C inkubiert. 1 ml vorgewärmtes (37°C) kappa 1776 Medium wurde dann mit einem solchen aliquoten Teil inokuliert und 1 h bei 37°C kultiviert. Zum Ausstreichen der Transformanden wurden die Zellen zentrifugiert (3000 Upm, 15 min, 4°C), in YT-Medium resuspendiert und auf Indikatorplatten ausgestrichen. Entsprechend der zu erwartenden Zahl von Transformanden wurde eine geeignete Menge der Suspension zum Ausstreichen verwendet.

Schnellanalytische Standard-Plasmidisolierung

Das beschriebene Verfahren stellt eine Modifizierung der Methode von H.C. Birnboim und J. Doly (1979), Nucleic Acids Res. 7, 1513; T. Maniatis et al (1982), Cold Spring Harbour Laboratory, Molecular Cloning dar. Für jeden zu analysierenden Transformanden wurde eine 2 ml Übernacht-Kultur hergestellt (37°C, 16 h, Rotationsrad). 1,5 ml der Kultur wurden 1 min bei 12.000 x g zentrifugiert. Das Pellet wurde in frisch zubereiteter Lösung von Lysozym-Mix aufgelöst und 5 min bei 20°C inkubiert.

Die Probe wurde im weiteren 5 min auf- Eis nach Zugabe von frisch hergestellter eiskalter 0,2 M NaOH, welche 1 % SDS enthält, inkubiert. Zur Ausfällung der chromosomalen DNA und der Proteine wurden 150 µl eiskaltes Kaliumacetat, pH 4,8, zugegeben. Nach 5-minütiger Inkubation auf Eis und 10-minütiger Zentrifugation bei 12.000 x g wurde der Überstand in ein frisches Reagensglas überführt und mit Sevag extrahiert. 500 µl Isopropanol wurden zur wäßrigen Phase zugegeben. Das Gemisch wurde dann 30 min bei -20°C inkubiert. Nach Zentrifugation (10 min, 12.000 x g) wurde das Pellet mit 80 %igem Ethanol gewaschen und kurz im Vakuum getrocknet.

Standard-DNA-Sequenzierung

Die Standard-DNA-Sequenzierung wurde ausgeführt, wie das im Protokoll der Hersteller (Richtlinien zur schnellen und einfachen Plasmidsequenzierung, Boehringer Mannheim (1986)) beschrieben wird.

Wachstum und Induktion von Bakterienstämmen

Bakterienkulturen wurden in Medien gezüchtet, die mit geeigneten Antibiotika ergänzt waren. Zur Erzielung der Expression von β-Galactosidase-Fusionsproteinen wurde E. coli RRI M15, transformiert mit dem Expressionsplasmid pES 44.1.1 oder pES 45.1.3, in 2 ml LB-Ampicillin-Medium inokuliert. Nach 12- bis 14-stündigem Wachstum bei 37°C in Schüttelkolben wurden 1 ml-Proben direkt zur Inokulierung von 100 ml LB-Ampicillin-Medium, welches 0,2 mMol IPTG enthielt, verwendet. Ein Klon, der pUR 278 ohne Aprotiningen-Insert enthielt, wurde als negative Kontrolle unter den gleichen Bedingungen kultiviert. Nach 12- bis 16-stündigem Wachstum bei 37°C unter Schütteln wurden die Zellen durch Zentrifugation bei 5.000 Upm (10 min) in einem Beckman JA 10-Rotor geerntet.

Standard-SDS-Polyacrylamidgel-Elektrophorese

Der Nachweis der Proteine erfolgte mit SDS-Polyacrylamid-Elektrophorese nach Laemmli, (1970), Nature, 277, S. 680 (s. auch B.D. Hames und D. Rickwood, 1981, Gel Electrophoresis of Proteins, IRL Press Limited, Oxford).

Ca. 1 x $10^9$ Zellen wurden zentrifugiert, in SDS-Probenpuffer aufgelöst (70 mM Tris, 10 % Glycerin, 1 % SDS, 5 % beta-Mercaptoethanol, 0,1 mM EDTA), 5 min bei 95 °C inkubiert und auf jeweils eine Bahn aufgegeben. Nach Elektrophorese wurden die Gele mit Coomassieblau R 250 angefärbt.

Bestimmung der Aminosäuresequenz

Ca. 0,5 bis 2 nMol des Proteins wurden in 30 µl TFA gelöst. Die Probe wurde auf ein Glasfaserfilter aufgegeben, welches mit 3 mg Polybren vorbehandelt war. Die Sequenzanalyse erfolgte mit Hilfe des Gasphasen-Protein-Sequenzierers von Applied Biosystems (Inc. USA) gemäß Hewick (R. M. Hewick, M. W. Hunkapiller, L. E. Hood, W. Dreger 1981, J. Biol. Chem. 256, 7990-7997). Die Aminosäure-Phenylthiohydantoin-Derivate, die in jedem Schritt freigesetzt wurden, wurden unter Verwendung einer Cyano-HPLC-Säule (DuPont) und einem Auftrennungssystem, wie von Beyreuther beschrieben, (K. Beyreuther, B. Biesler, J. Bowens, R. Dildrop, K. Neufer, K. Stüber, S. Zais, R. Ehring, P. Zabel (1983), Modern Methods in Protein Chemistry, 303-325, Walter de Gruyter + Co., Berlin), analysiert. Ein Waters-HPLC-System, mit einer M 510 Pumpe, einem WISP 710B Autoinjektor, einem LC-Spektrophotometer M 481 und einem Shimadzu-Integrator C-R3A, wurde verwendet.

Säurehydrolyse und Aminosäureanalyse

Ca. 1 nMol des Proteins wurden in ein Pyrex-Reagensglas gegeben, zu welchem 200 µl 6M HCl (konstant siedende HCl), die 0,05 % 2-Mercaptoethanol (I. T. Potts jr. 1969, Anal. Biochem. 131, 1-15) enthielt, zugefügt wurden. Die Reagensgläser wurden unter Vakuum versiegelt und 22 h bei 110°C inkubiert. Die Hydrolysate wurden schnell getrocknet, in 150 µl 0,2 M Natriumcitratpuffer pH 2,2 aufgelöst unf filtriert. Die Aminosäureanalyse wurde mit einem Biotronic LC 5000 Aminosäureanalysator, ausgestattet mit einem Fluoreszenzdetektor und einem Shimadzu C-R2AX-Integrator, durchgeführt. Die Aminosäuren wurden nach Reaktion mit o-Phthal-dialdehyd quantitativ bestimmt, und zwar im wesentlichen, wie dies von Benson (J. R. Benson, P. E. Hare 1975, Proc. Natl. Acd. Sci. USA 72, 619-622) beschrieben wurde.

Inhibierungsassays für Leukozytenelastase

Leukozytenelastase wurde bestimmt, wie dies von K. Nakajima et al (1979), J. Biol. Chem. 254, 4027 beschrieben ist. Die Bestimmungen wurden unter folgenden Bedingungen durchgeführt:

| | |
|---|---|
| Substrat (Stammlösung) | 0,1 M Methoxysuccinyl-L-alanyl-L-alanyl-L-prolyl-L-valin-p-nitro-anilid in Dimethylformamid. Die Stammlösung wurde bei -18°C gelagert. |
| Substrat (Menge pro test) | 6,5 µl |
| Substrat-Hersteller | Bachem Bubendorf / Schweiz |
| Enzym (Stammlösung) | 0,01 mg/ml humane Leukozytenelastase in 50 % Ethylenglykol. Die Lösung wurde bei -18°C gelagert. |
| Enzym (Menge pro Test) | 5 µl |
| Enzym-Hersteller | Elastin Products Company Pacific / USA |
| Puffer | 0,2 M Tris/HCl, pH 8,0 + 0,05 % Tween 80 |

Allgemeines Verfahren

Die Inhibitorprobe wurde mit einer Puffermenge verdünnt, so daß das Endvolumen (nach Zugabe des Substrats) 0,65 ml betrug. Daraufhin wurde das Enzym zugegeben, und das Gemisch wurde 30 min bei Raumtemperatur stehen gelassen. Schließlich wurde die Substratlösung zugegeben und die Zunahme der Extinktion bei 405 nm im folgenden automatisch für jede Probe registriert. Die lineare Zunahme der Extinktion während der ersten 10 min ($\Delta$ OD) wurde als Maß für die Enzymaktivität angesehen.

Zur Bestimmung der Inibitionswirkung wurde die Enzymaktivität mit und ohne Zugabe von Inhibitoren gemessen. Der Inhibierungsgrad in % wurde wie folgt berechnet:

$$\text{Inhibierung (\%)} = 100 \times (1 - \frac{OD_{\text{Probe mit Inhibitor}}}{OD_{\text{Probe ohne Inhibitor}}})$$

Bestimmung der $K_i$-Werte aus den Titrationskurven von humaner Leukozytenelastase mit den Inhibitoren

Zu einer Reihe von Lösungen, die ca. 200 ng humane Leukozytenelastase in 900 ul 0,2 M Tris-Puffer pH 8,0 mit jeweils 0,05 % Tween 80 enthielten, wurden verschiedene Inhibitormengen gegeben. Die Gemische wurden mindestens 2 h nach Auffüllen des Volumens auf 985 µl mit Testpuffer bei Raumtemperatur gehalten. Dann wurden 15 µl eines Gemisches, welches sich aus 10 µl Substrat-Stammlösung - 59 mg MeOSuc-Ala-Ala-Pro-val-pNA pro 1 ml Dimethylsulfoxid - zusammensetzt und 990 µl Testpuffer nach 5-minütiger Equilibrierung auf 30°C in einem thermostatisierten Küvettenhalter zu jeder Probe zugegeben und die Zunahme der Extinktion bei 405 nm bestimmt. Die $K_i$-Werte wurden nach M.W. Empie und M. Laskowski jr., Biochemistry 21, 2274-2284 (1982) unter Anwendung der folgenden Gleichung berechnet:

$$K_i = \frac{[E_f] \, [I_f]}{[EI]}$$

In dieser Gleichung stellen $[E_f]$ und $[I_f]$ die molaren Konzentrationen von jeweils nicht-komplexiertem Enzym und nicht-komplexiertem Inhibitor dar; $[EI]$ ist die molare Konzentration des Enzym-Inhibitor-Komplexes.

Beispiele

Beispiel 1

Synthese und Reinigung von DNA-Fragmenten, die für Val-15-Aprotinin-Varianten kodieren

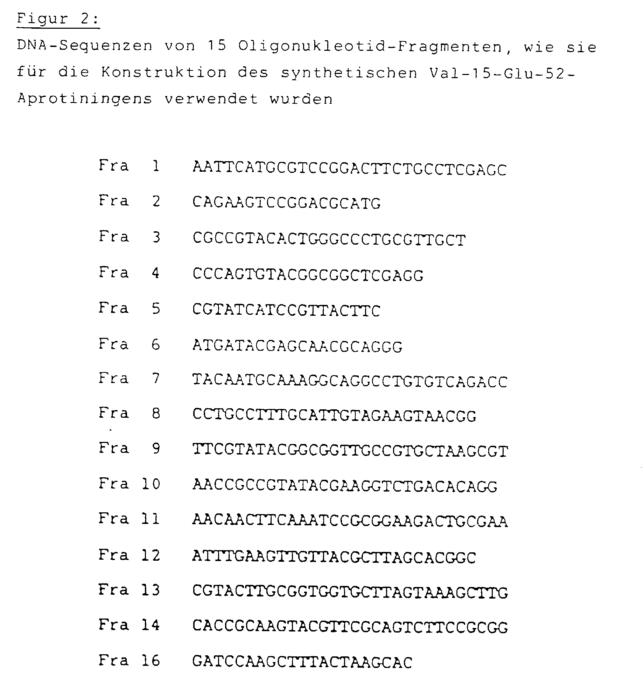

Die Oligonukleotide, die das Gen umfassen (s. Fig. 2), wurden unter Anwendung von Fest-Phasen-Synthese-Methoden hergestellt. Das Syntheseschema für die Oligomeren sieht die Verwendung von Protonen-aktivierten, geschützten 2'-Desoxyrihonukleotid-phosphoramiditen vor. Sämtliche Sequenzierungsschritte wurden in automatisierter Weise auf einem Applied Biosystems Modell 380 DNA-Synthesizer unter Verwendung geschützter Nukleotide, Lösungsmittel, Chemikalien und Reagenzien, die vom Hersteller erhalten wurden, durchgeführt. Der Festphasenträger, vom selben Hersteller, stellte kontrolliertes Porenglas dar, an welchem bereits das Ausgangs-3'-Nukleotid haftete. In Übereinstimmung mit den Verfahrensinstruktionen der Hersteller und den Anwender-Bulletins wurden bestimmte Modifikationen im automatisierten Reaktionszyklus eingeführt. Nach Beendigung der Synthese wurden die Oligomeren entblockt und von dem festen Träger im DNA-Synthesizer entsprechend den Anleitungen der Hersteller abgespalten.

Die Entfernung der Blockierungsgruppen erfolgte im weiteren durch Erwärmen der wäßrigen Lösung, die das Oligomer enthielt, mit konzentriertem Ammoniumhydroxid über 4 bis 24 h bei 55°C in einer versiegelten Phiole. Die erhaltene Lösung wurde eingedampft, der Rückstand in 0,01 M Triethylammonium-bicarbonat-puffer, pH 7,0 (TEAB-Puffer) aufgelöst. Diese Lösung wurde über Sephadex-G 50$^{(R)}$-Gelfiltrationsharz chromatographiert. Diese Säule wurde in dem gleichen TEAB-Puffer hergestellt und mit demselben eluiert. Das mit dem Leervolumen eluierende Material wurde vereinigt und die Lösung eingedampft.

Ein Teil des Rückstandes (10 bis 40 % der Extinktionseinheiten bei 260 nm), aufgelöst in einem Aufgabepuffer (Zusammensetzung: 0,1 % Bromphenol Blau, 0,1 % Xylolcyanol, 10 mMol Dinatrium-EDTA,in Formamid) wurde durch Elektrophorese auf Polyacrylamidgelen weiter gereinigt. Die Gelgröße betrug 18 x 32 cm mit einer Dicke von 1,5 mm. Die Vertiefung für jedes auf diese Weise gereinigte Oligomer war 2 bis 5 cm breit; es wurden bis zu 5 Oligomere auf einem einzigen Gel gereinigt. Die Konzentration des Acrylamids in dem Gel variierte von 14 bis 20 %, je nach der Kettenlänge des gewünschten Produktes. Bei längeren Oligomeren ist ein 14 %iges Acrylamidgel bevorzugt, während kürzere Oligomere auf bis zu 20 %igem Acrylamidgel gereinigt wurden. Die Gele enthielten auch 7 M Harnstoff und Tris-Borat-EDTA-Puffer (0,1 M Tris, 0,1 M Borat, 0,2 mM EDTA, pH 8,3). Der Laufpuffer war das gleiche Tris-Borat-EDTA-Gemisch. Elektrophorese wurde bei 20 bis 60 Watt, konstantem Strom, über 18 bis 6 h durchgeführt. Derartige standardisierte Methoden werden in verschiedenen "User Information Bulletins", die von Applied Biosystems erhältlich sind, beschrieben.

Nach Beendigung der Elektrophorese wird das Gel in einer plastischen Umhüllung aufgenommen, und die Oligomere werden durch Bestrahlen mit ultraviolettem Licht sichtbar gemacht. Dieses Sichtbarmachen erfolgt, indem man das verpackte Gel auf eine Platte für die fluoreszierende Dünnschichtchromatographie gibt und sich das Gel mit einer kurzwelligen UV-Lichtquelle besieht. Mit Hilfe dieses Verfahrens zur Sichtbarmachung erscheint das gewünschte Produkt als das am langsamsten wandernde, hauptsächlich blaue DNA-Fragment. Die gewünschte Bande wird aus dem Gel herausgeschnitten. Das DNA-Oligomer wird aus der Gelschnitte auf pulverförmige Diethylaminoethyl(DEAE)-cellulose unter Verwendung eines EpiGene D-Gel$^{(R)}$-Elektrophoreseapparates eluiert. Das Oligomer wird aus der Cellulose durch Elution mit 1 M TEAB-Puffer gewonnen. Die das Oligomer enthaltende Pufferlösung wird eingedampft, der Rückstand in 0,01 M TEAB-Puffer aufgelöst und dann entsalzt, indem er über eine vorstehend beschriebene Sephadex-G 50 -Säule geleitet wird. Das im Leervolumen eluierende Material wird vereinigt und unter Erhalt des Endproduktes lyophilisiert.

Mit Hilfe der vorstehend beschriebenen Verfahren wurden ca. 0,5 bis 5,0 $A_{260}$-Einheiten von jedem der gereinigten Oligomere erhalten.

Beispiel 2

Konstruktion eines synthetischen Mastergens für Val-15-Glu -52-Aprotinin und Insertion in das Plasmid pUC8 unter Erhalt von Plasmid pRK 54.1.1

Das Konstrukt des Mastergens ist in Fig. 1 wiedergegeben. Es setzt sich aus den Baublöcken alpha, beta, gamma und delta zusammen und wird durch Zusammensetzen der in Fig. 2 gezeigten 15 gereinigten Oligonukleotide erhalten. Die DNA-Sequenz, die aus Fig. 3 hervorgeht, umfaßt das Initiierungscodon ATG, zwei Terminierungscodons, TAG und TAA, die terminalen Restriktionsstelle Eco RI, Hind III und Bam HI und interne Restriktionsstellen. Die Wahl dieser Stellen erleichterte die Klonierung der Kodierungssequenz und deren Modifizierung.

Die zur Herstellung dieses synthetischen Gens verwendete Konstruktion bedarf neben der Fragmente auch dem Einsatz von Polynukleotid-kinase, T4-DNA-Ligase und Restriktionsenzymen, wie dies unter Materialien und Methoden im Detail beschrieben ist.

Fünfzehn gereinigte Oligonukleotid-Fragmente wurden in 50 mM TEABC (Triethylammonium-bicarbonat-puffer, pH 7,5) mit einer Endkonzentration von 10 pMol/µl aufgelöst. Die Phosphorylierung sämtlicher Fragmente erfolgte in vier separaten Teilen (Frag. 1, 3; Frag. 2, 4, 6; Frag. 5, 7, 9, 11, 13; Frag. 8, 10, 12, 14, 16). Für präparative Zwekke wurden jeweils 80 pMol eines jeden Fragmentes in einem Gemisch von 1X PNK-Mix, 2 µM ATP, 0,5 µCi 32 gammaATp pro 10 pMol Fragment, 10 Einheiten PNK pro pMol Fragment, aufgelöst, so daß folgende Gesamtvolumen erhalten wurden: Für Frag. 1, 3: 300 µl; für Frag. 2, 4, 6: 400 µl für Frag. 5, 7, 9, 11, 13; und Frag. 8, 10, 12, 14, 16: 700 µl. Die Umsetzung jeder Teilsequenz erfolgte 30 min bei 37°C. Sämtliche Teilsequenzen wurden phenolisiert, mit Ethanol ausgefällt, gewaschen und getrocknet.

Für Hybridisierungszwecke wurden Frag. 1, 3 und Frag. 2, 4, 6 (Block A) aufgelöst und in 1X Ligase-Mix bei einem Gesamtvolumen von 120 µl gemischt, 5 min bei 70°C inkubiert, innerhalb von 5 h auf Raumtemperatur abgekühlt. Die anderen Fragmente (Block B) wurden in 240 µl nach dem gleichen Verfahren hybridisiert.

Für Ligierungszwecke wurde die Lösung von Block A mit 12 µl 10 mM ATP, 12 µl 100 mM DTE, 20 µl T4-DNA-Ligase ergänzt; die Lösung von Block B wurde mit dem Zweifachen ergänzt. Die Umsetzung erfolgte 7 h bei 14°C. Nachdem zu Block A 10 µl T4-DNA-Ligase und zu Block B 20 µl zugegeben worden waren, wurde erneut 45 min bei 14°C inkubiert. Diese Gemische wurden phenolisiert, mit Ethanol ausgefällt und getrocknet.

Der erhaltene Block A wurde in 90 µl 1X SP-100 und 10 µl Eco RI (10 E/µl), Block B in 90 µl 1X SP-50 und 10 µl Bam HI aufgelöst und 1 h bei 37°C inkubiert. Die Reaktionen wurden durch Phenolextraktion und Ethanolfällung gestoppt; es wurde eine Gelelektrophorese auf einem 6 %igen Polyacrylamidgel durchgeführt, und die DNA-Blöcke wurden nach dem gleichen Verfahren, wie dies in Beispiel 1 beschrieben ist, gewonnen.

Gleiche Mengen an radioaktiv markiertem Block A und B wurden in Wasser aufgelöst, auf 1X Ligase-Mix eingestellt und - wie vorstehend beschrieben - zur endgültigen Ligierung zu einem synthetischen Gen hybridisiert. Dazu wurden 3 µl 10 mM ATP, 3 ul 100 mM DTE, 3 µl T4-DNA-Ligase zu 22 µl Hybridisierungsgemisch zugegeben und 7 h bei 14°C inkubiert. Es wurde erneut 1 µl T4-DNA-Ligase zugegeben und die Reaktion 45 min bei 14°C fortgesetzt. Das Ligierungsprodukt wurde durch Phenolextraktion und Ethanolfällung gereinigt. Es wurde eine Standard-Restriktionsenzym-Spaltung (Bam HI 1,5 µl, Eco RI 1,5 µl Doppelspaltung) in SP-50 durchgeführt. Das Material wurde mit Phenol extrahiert; vor der Ethanolfällung wurde die wäßrige Lösung auf 3 mM MgCl$_2$, 0,3 M Natriumacetat, eingestellt. Dann wurde eine Elektrophorese auf 6 %igem Polyacrylamidgel durchgeführt, und das Gen nach dem gleichen Verfahren, wie dies in Beispiel 1 beschrieben ist, gewonnen.

Das synthetische Val-15-Glu-52-Aprotinin-Mastergen, das nach diesem Verfahren erhalten wurde, wurde in einen mit EcoRI/Bam HI gespaltenen pUC8-Vektor (J. Vieira und J Messing, Gene 19 259 (1982)) wie folgt insertiert:

Gereinigte pUC8 DNA (ca. 30 pMol) wurde zweimal mit EcoRI und Bam HI unter Standard-Restriktionsendonuklease-Spaltbedingungen gespalten, wobei ein kleines internes EcoRI-Bam HI-Fragment herausgespalten wurde. Diese Präparation wurde mit Phosphatase aus Kälberdarm entphosphoryliert, durch Agarosegel-Elektrophorese aufgetrennt; das große EcoRI-Bam HI-Fragment des Vektors wurde gereinigt (Standardbedingungen).

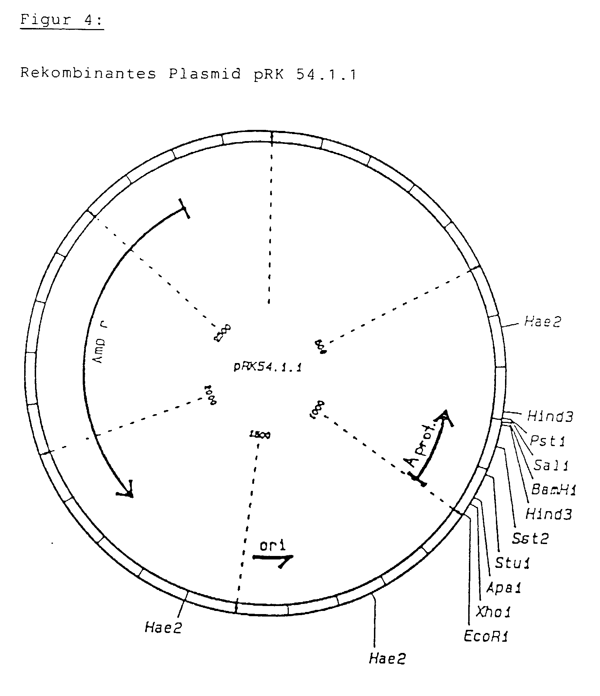

Die Konstruktion von pRK 54.1.1 (siehe Fig. 4) erfolgte durch Ligieren der Gesamtmenge an gereinigtem synthetischen Aprotiningen mit 1 pMol Vektor (1,8 Einheiten T4-DNA-Ligase, 1X Ligase-Mix, Gesamtvolumen 45 µl, 7-stündige Inkubation bei 14°C, Zugabe von 1 Einheit T4-DNA-Ligase und 45-minütige Reinkubation bei 14°C).

Unter Anwendung des Transformierungsverfahrens nach D. Hanahan wurde E.coli Stamm RRI delta M15 (A. Kalnins et al (1983), EMBO Journal 2, 593, ATCC 35102) als Wirtzelle verwendet. Es wurden 15 "weiße" Transformanden auf Indikatorplatten, die 200 µg/ml Ampicillin enthielten, erhalten. Alle 15 Transformanden wurden unter Anwendung einer Modifizierung der schnellanalytischen Plasmid-Isolierungsmethode nach Birnboim und Dcly 1979 einem Screening unterworfen. Dazu wurden Pellets der 15 Proben in 30 µl 1X SP-100, welches 1 µg RNase A enthält, erneut aufgelöst. Es wurde eine Restriktionsspaltung mit Eco RI und Bam HI durchgeführt.

Nach Gelelektrophorese wurde festgestellt, daß vier der fünfzehn Transformanden Plasmid-DNA enthielten, die ein ca. 200 Basenpaare langes Eco RI-Bam HI-Fragment aufwies. Alle Transformanden, die dieses Eco RI-Bam HI-Fragment aufwiesen, wurden im großen Maßstab gezüchtet und die Plasmide eines jeden isoliert und weiter analysiert. Zwei derselben wurden nach dem Standard-Sequenzierungsverfahren, wie es in Materialien und Methoden beschrieben ist, sequenziert; alle wiesen die Sequenz des Val-15-Glu-52-Aprotiningens auf.

Beispiel 3

Rekombinante Plasmide pNH 02.1.1. (Val-15-Leu-17-Glu-52-Aprotinin), pNH 16.1.1. (Val-15-Leu-17-Glu-39-Glu-52-Aprotinin), pRK 126.1.24 (Val-15-Leu-17-Aprotinin) und pRK 113.1.1. (Val-15-Leu-17-Thr-52-Aprotinin)

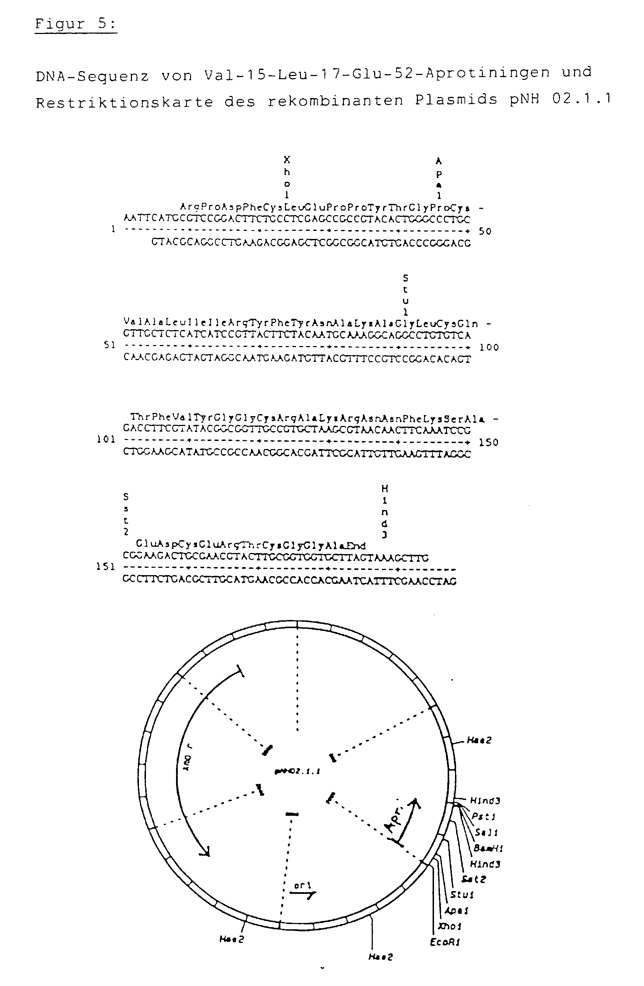

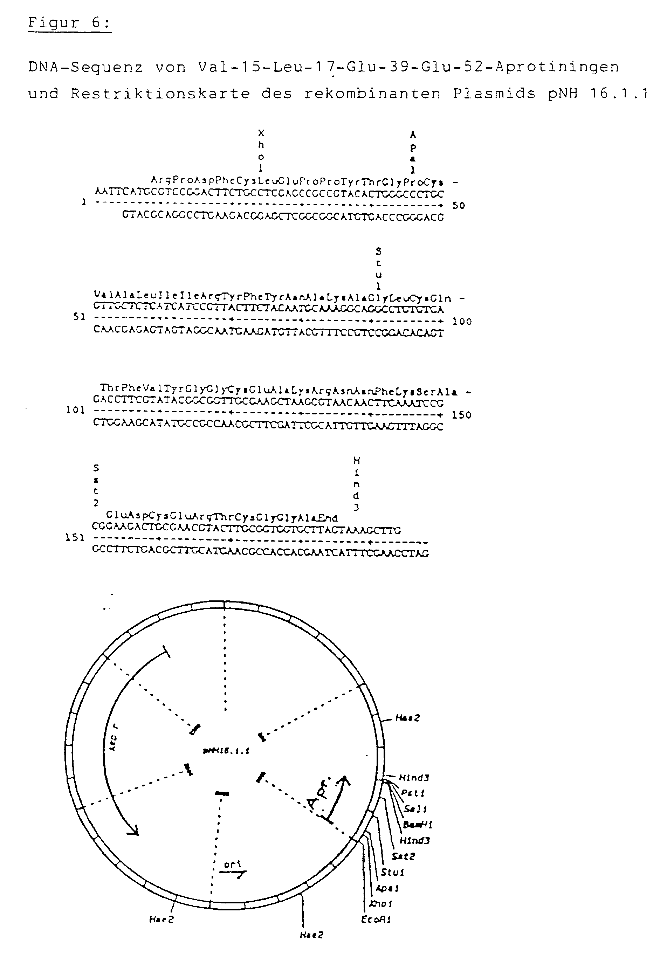

Das rekombinante Plasmid pNH 02.1.1. (Fig. 5) enthält einen Kodierungsbereich für das synthetische Val-15-Leu-17-Glu-52-Aprotiningen. Das rekombinante Plasmid pNH 16.1.1. (Fig. 6) enthält einen Kodierungsbereich für das synthetische Val-15-Leu-17-Glu-39-Glu-52-Aprotiningen.

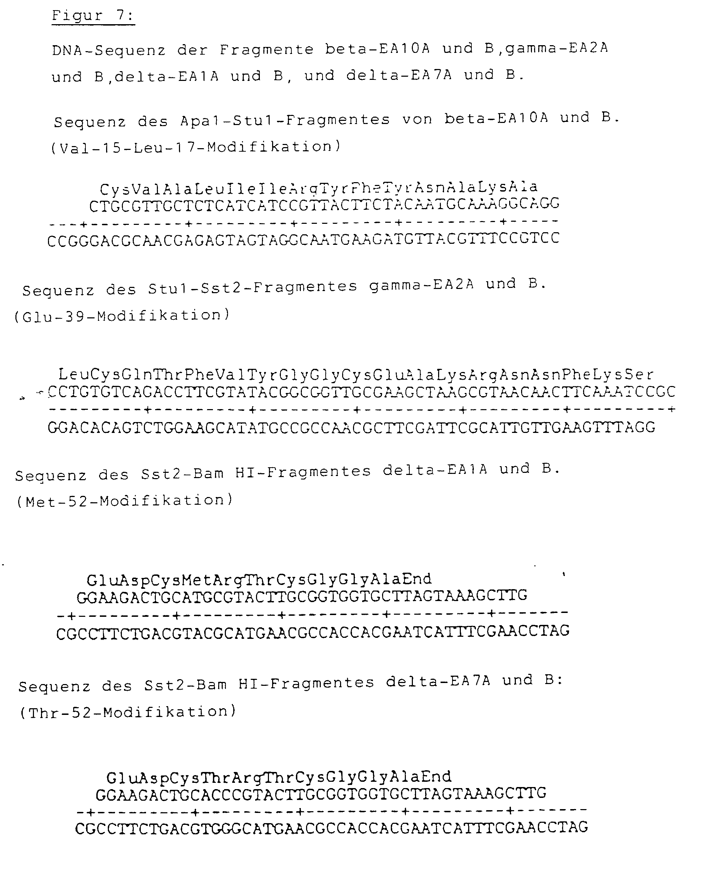

Das Plasmid pNH 02.1.1. (Val-15-Leu-17-Glu-52-Aprotinin) wurde durch Austausch eines beta-Blockes, der ein Apa I - Stu I-Fragment darstellt und ein Codon für Leu in Position 17 anstelle von Arg (see Fig. 7) enthält, erhalten.

Ca. 100 pMol der synthetischen ss DNA-Fragmente beta-EA10A und beta-EA10B (Fig. 7) wurden in 20 µl Wasser aufgelöst, 5 min auf 95°C erwärmt und langsam (5 h) auf Raumtemperatur abgekühlt. Das hybridisierte, unphosphorylierte Fragment wurde mit 1,5 pMol gereinigter DNA aus pRK 54.1.1, welcher das Apa I - Stu I-Fragment fehlte, ligiert.

Die Transformierung von E.coli RRIΔM15 erfolgte mit 50 % des Ligierungsgemisches. Von 1.500 Transformanden wurden 24 durch analytische Plasmidisolierung und Restriktionsanalyse getestet.

Sämtliche waren positiv; zwei davon wurden, wie dies in Materialien und Methoden beschrieben ist, sequenziert. Der Transformand pNH 02.1.1. wurde für die weiteren Versuche verwendet.

Das Plasmid pNH 16.1.1, welches das Val-15-Leu-17-Glu-39-Glu-52-Aprotiningen enthielt, wurde durch einfachen Austausch eines gamma-Blockes, der ein Stu I - Sst II- Fragment von pNH 02.1.1 darstellt, und ein Codon für Glu in Position 39 anstelle von Arg enthält, erhalten.

Ca. 100 pMol der synthetischen ss DNA-Fragmente gamma-EA 2A und gamma-EA 2B (Fig. 7) wurden in 20 µl Wasser aufgelöst, 5 min auf 95°C erwärmt und langsam (5 h) auf Raumtemperatur abgekühlt. Das hybridisierte und unphosphorylierte Fragment wurde mit 1,5 pMol gereinigter DNA aus pNH 02.1,1, welcher das Stu I - Sst II-Fragment fehlte, ligiert.

Die Transformierung von E.coli RRI Δ M15 erfolgte mit 50 % des Ligierungsgemisches. Die E.coli-Transformanden wurden durch analytische Plasmidisolierung und Restriktionsanalyse getestet. Ein positiver Klon wurde, wie dies in Materialien und Methoden beschrieben ist, sequenziert. Der Transformand pNH 16.1.1 wurde für die weiteren Experimente verwendet.

Das Plasmid pRK 126.1.24 (Val-15-Leu-17-Met-52-Aprotinin) wurde aus pNH 02.1.1 durch Austausch eines delta-Blockes, der das Codon für Threonin in Position 52 anstelle von Glu enthielt, erhalten. Ca. 100 pMol der synthetischen ss DNA-Fragmente delta-EA 1A und delta-EA 1B (Fig. 7) wurden in 20 µl Wasser aufgelöst, 5 min auf 95°C erwärmt und langsam (5 h) auf Raumtemperatur abgekühlt. Das hybridisierte, unphosphorylierte Fragment wurde mit 1,5 pMol gereinigter DNA aus pNH 02.1.1, welcher das Sst II - Bam HI-Fragment fehlte, ligiert.

Die Transformation von E.coli RRI M15 erfolgte mit 50 % des Ligierungsgemisches. Die E.coli Transformanden wurden mit Hilfe der analytischen Plasmidisolierung und Restriktionsanalyse getestet. Ein positiver Klon wurde, wie dies in Materialien und Methoden beschrieben ist, sequenziert. Der Transformand pRK 126.1.24 wurde für die weiteren Versuche eingesetzt.

Das Plasmid pRK 113.1.1 (Val-15-Leu-17-Thr-52-Aprotinin) wurde aus pNH 02.1.1 durch Austausch eines delta-Blockes, welcher das Codon für Threonin in Position 52 anstelle von Glutaminsäure enthielt, erhalten. Ca. 100 pMol der synthetischen ss DNA-Fragmente delta-EA7A und delta-EA7B (Fig. 7) wurden in 20 µl Wasser aufgelöst, 5 min auf 95°C erwärmt und langsam (5 h) auf Raumtemperatur abgekühlt. Das hybridisierte, unphosphorylierte Fragment wurde mit 1,5 pMol gereinigter DNA aus pNH 02.1.1, welcher das SstII/Bam HI-Fragment fehlte, ligiert. Die Transformation von E.coli RRIΔ M15 erfolgte mit 50 % des Ligierungsgemisches. Die E.coli Transformanden wurden durch analytische Plasmidisolierung und Restriktionsanalyse getestet. Ein positiver Klon wurde, wie dies in Materialien und Methoden beschrieben ist, sequenziert. Der Transformand pRK 113.1.1 wurde für die weiteren Experimente verwendet.

Beispiel 4

Expressionsplasmide pES 044.1.1, pES 045.1.3, pNH 05.1.1 und pNH 21.1.1

Ein Beispiel zur Expression der Gene von Aprotininvarianten in E.coli wird im folgenden angegeben, und zwar durch Expression der genannten Gene als Fusionen mit dem lacZ-Gen (U. Rüther und B. Müller-Hill (1983), EMBO Journal, 2, 1791-1794) oder mit dem N-terminalen Teil von RNA-Polymerase des Phagen MS-2 (Remault et al (1981), Gene 15, 81-93).

Die aus den Plasmiden pNH 02.1.1 (Val-15-Leu-17-Glu-52-Aprotinin) und pNH 16.1.1 (Val-15-Leu-17-Glu-39-Glu-52-Aprotinin) erhaltenen Aprotiningene wurden in das Expressionsplasmid pUR 278 ligiert (U. Rüther und B. Müller-Hill (1983), EMBO Journal, 2, 1791-1794).

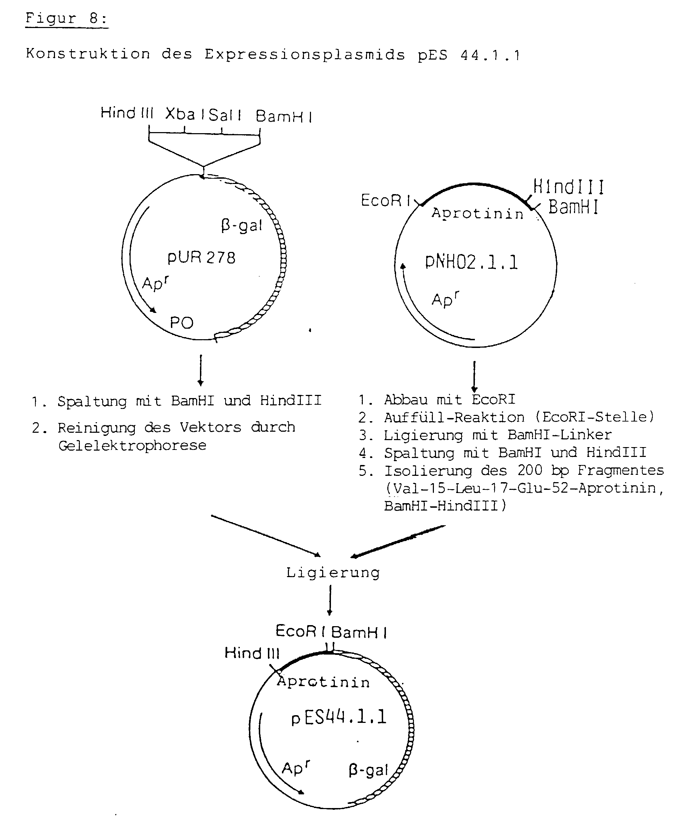

Zur Klonierung des synthetischen Aprotiningens im Expressionsvektor pUR 278 wurden die Klonierungsstellen Bam HI und Hind III gewählt. Aus diesem Grunde war es notwendig, das Aprotiningen durch Zufügen einer Bam HI-Stelle am 5'-EcoRI-Ende des Gens und unter Verwendung der Hind III-Stelle am 3'-Ende zu modifizieren (s. Fig. 8).

5 pMol des Plasmids pNH 02.1.1 wurden 5 h lang bei 37°C vollständig mit EcoRI (1,5 pMol/µl) in 50 µl 1X SP-100 gespalten.

Nach Spaltung wurde das Material mit Phenol extrahiert, mit Ethanol ausgefällt und unter Vakuum getrocknet.

Die überstehenden 5'-EcoRI-Enden dieses Materials wurden enzymatisch mit DNA-Polymerase I (Klenow-Fragment), dATP und TTP aufgefüllt.

5 pMol dieser DNA wurden in 2 µl 10 mM dATP, 2 µl 10 mM TTP, 5 µl 10 x NT-Puffer und 39 µl Wasser gelöst.

Dann wurden 2 µl DNA-Polymerase I (Klenow-Fragment, 5 Einheiten/µl) zugegeben, und es erfolgte Inkubation (30 min) bei Raumtemperatur.

Das Material wurde mit Phenol extrahiert, mit Ethanol ausgefällt, zweimal mit 80 %igem Ethanol gewaschen und in 20 µl TE-Puffer aufgelöst. 20 µl dieses Materials mit glatten Enden dienten zur Ligierung mit Bam HI-Linker. Dazu wurden 200 pMol phosphorylierter Bam HI-Linker an 10 pMol DNA-Enden ligiert (Standard-Ligierungsbedingungen, 4,5 µl T4-DNA-Ligase, Gesamtvolumen 60 µl), und 18 h bei 14°C inkubiert. Das Reaktionsgemisch wurde mit Phenol/ Sevag extrahiert, mit Ethanol gefällt, gewaschen, getrocknet und in 10 µl TE aufgelöst.

Zur Herstellung des synthetischen Aprotiningens mit Bam HI-und Hind III-Termini, wurde das "mit Linker versehene" lineare Plasmid (10 pMol) zunächst mit Hind III und dann mit Bam HI unter Standardbedingungen gespalten. Das Fragment wurde nach Auftrennung durch 1,8 %ige Agarosegelelektrophorese isoliert und sorgfältig gereinigt (s. Standardverfahren).

### Vektorherstellung

Der Stamm-Vektor pUR 278 (ca. 5 pMol) wurde zunächst mit Hind III (Standardbedingungen) gespalten, durch Phenol/ Sevag-Extraktion und Ethanolfällung gereinigt, wieder aufgelöst und dann mit Bam HI (Standardbedingungen) gespalten. Dieses Material wurd auf ein 1 %iges Agarosegel geschichtet, einer Elektrophorese unterworfen, isoliert und entsprechend den Standardbedingungen gereinigt, um es von dem 18 Basenpaar langen Bam HI - Hind III-Fragment zu befreien, welches bei der Ligierung mit dem synthetischen Aprotiningen kompetieren würde.

### Ligierung und Transformation

Zur Ligierung wurden 0,3 pMol Vektor, 1,5 pMol Fragment (ungefähr), 2 Einheiten T4-DNA-Ligase verwendet (Standardbedingungen, Gesamtvolumen 30 µl, Inkubation 4 h bei 14°C).

Die Transformation erfolgte mit E.coli Stamm RRI delta M15 als Wirt unter Verwendung eines Drittels des Ligierungsgemisches (Standardbedingungen). Insgesamt wurden 173 "blaue" Kolonien auf Indikatorplatten, die 200 µg Ampicillin/ml enthielten, erhalten. Davon wurden 12 Transformanden weiter durch schnellanalytische Plasmidisolierung (Standardbedingungen) analysiert. Von den 173 Transformanden sollten 30 background-Transformanden darstellen, wie dies aufgrund des Prozentsatzes der Transformanden, die durch Vektorligierung erhalten wurden, errechnet wurde. Dieses Ergebnis wurde durch Restriktionsanalyse der Plasmide der 12 Transformanden bestätigt. 8 davon waren positiv, indem sie ein Bam HI - Hind III-Restriktionsfragment von ca. 200 Basenpaaren aufwiesen. Die positiven rekombinanten Plasmide wurden auch mit Sst II, einer einzigen Restriktionsstelle im Aprotiningen, linearisiert. Die Basensequenzanalyse nach dem Standardverfahren, wie es in Materialien und Methoden beschrieben ist, zeigte, daß das Plasmid pES 44.1.1 das gewünschte Aprotinin-DNA-Fragment als Insertion enthielt (s. Fig. 8). Das Plasmid pES 44.1.1 wurde für die weitere Analyse und Expressionsarbeit verwendet. Die Konstruierung von Plasmid pES 45.1.3 erfolgte nach genau dem gleichen Verfahren unter Verwendung von Val-15-Leu-17-Glu-39-Glu-52-Aprotiningen aus pNH 16.1.1. Das positive rekombinante Plasmid pES 45.1.3 zeigte die korrekte DNA-Sequenz; dieses Konstrukt wurde für die weitere Analyse und Expressionsarbeit verwendet.

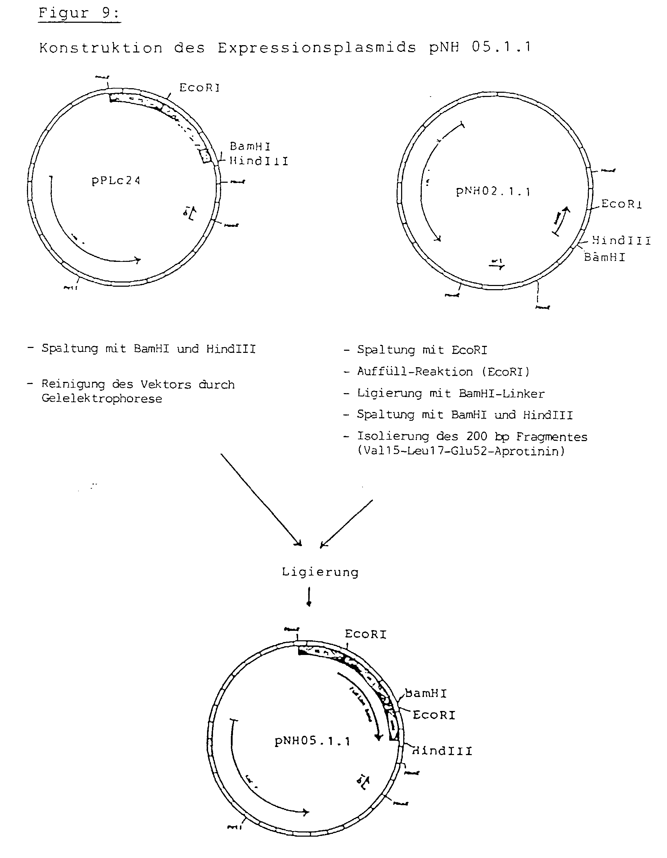

Die Konstruierung des Plasmids pNH 05.1.1 (Val-15-Leu-17-Glu-52-Aprotinin (s. Fig. 9) und pNH 21.1.1 (Val-15-Leu-17-Glu-39-Glu-52-Aprotinin) erfolgte unter Anwendung des Verfahrens, wie es in Fig. 9 aufgezeigt ist. Das Val-15-Leu-17-Glu-52-Aprotiningen wurde aus pNH 02.1.1 als EcoRI - Hind III-Fragment erhalten. Zur Klonierung im Expressionsvektor pPLc 24 war es erforderlich, das Aprotiningen durch Zufügen einer Bam HI-Stelle am 5'-EcoRI-Ende des Gens zu modifizieren. Dies erfolgte, wie es vorstehend für die Klonierung des Gens in pUR278 beschrieben ist. Das modifizierte Gen wurde in den Vektor pPLc 24 insertiert, welcher mit Bam HI und Hind III restriktiert worden war. pPLc 24 wurde von Prof. W. Fiers, Universität Gent, Belgien, erhalten. In diesem Konstrukt ist das Aprotiningen im Leseraster mit dem N-terminalen Teil der RNA-Polymerase des Phagen MS-2 verbunden (Remault et al, Gene 15, 81-93 (1981)). Das Plasmid pNH 05.1.1 wurde für die weitere Analyse und Expression verwendet.

Das Plasmid pNH 21.1.1 wude auf die gleiche Weise wie pNH 05.1.1 erhalten, nämlich durch Ligieren des modifizierten EcoRI/Hind III-Fragmentes, welches aus Plasmid pNH 16.1.1 erhalten wurden war, in Plasmid pPL 24, welches durch Bam HI und Hind III restriktiert worden war.

### Beispiel 5

### Konstruktion des alpha-Amylase-Sekretionsvektors pCH 2742

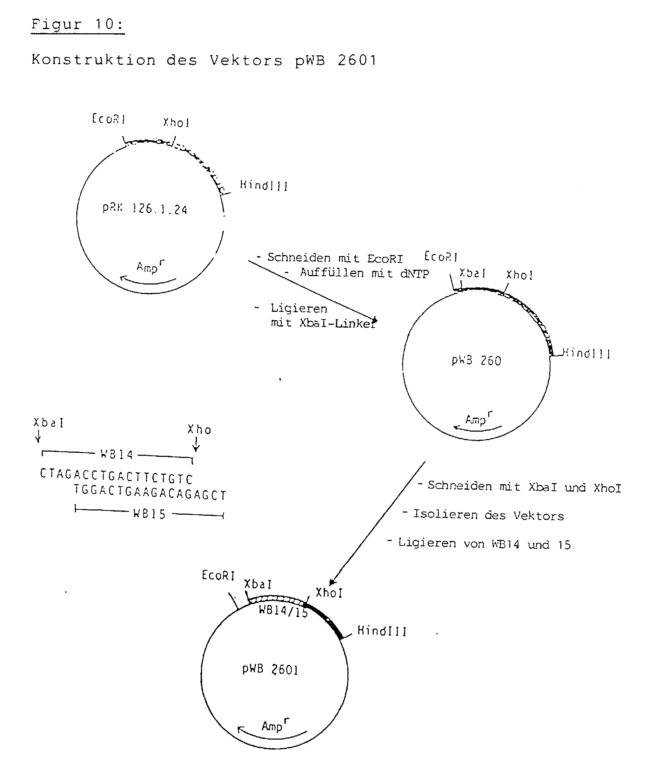

Zum Klonieren in E.coli Sekretionsvektor pCH 237 wurde das Gen für Val-15-Leu-17-Met-52-Aprotinin durch Einführung einer Xba I-Stelle am Codon für Arg-1 modifiziert. Zu diesem Zweck wurde pRK 126.1.24 an der EcoRI-Stelle restriktiert, und nach Auffüllen mit dNTP in Gegenwart von DNA-Polymerase (großes Fragment) wurde ein XbaI-Linker (Biolabs 1010) in die Restriktionsstelle ligiert (s. Fig. 10), wodurch das Plasmid pWB 260 erhalten wurde. pWB 260 wurde an Xbal und Xhol restriktiert, der Vektor wurde isoliert und ein Linker, der aus zwei synthetischen DNA-Fragmenten (WB 14 und WB 15) bestand, wurde in pWB 260 ligiert, wobei pWB 2601 erhalten wurde (Fig. 10).

Die Konstruktion des alpha-Amylase-Sekretionsvektors. wurde wie folgt ausgeführt:

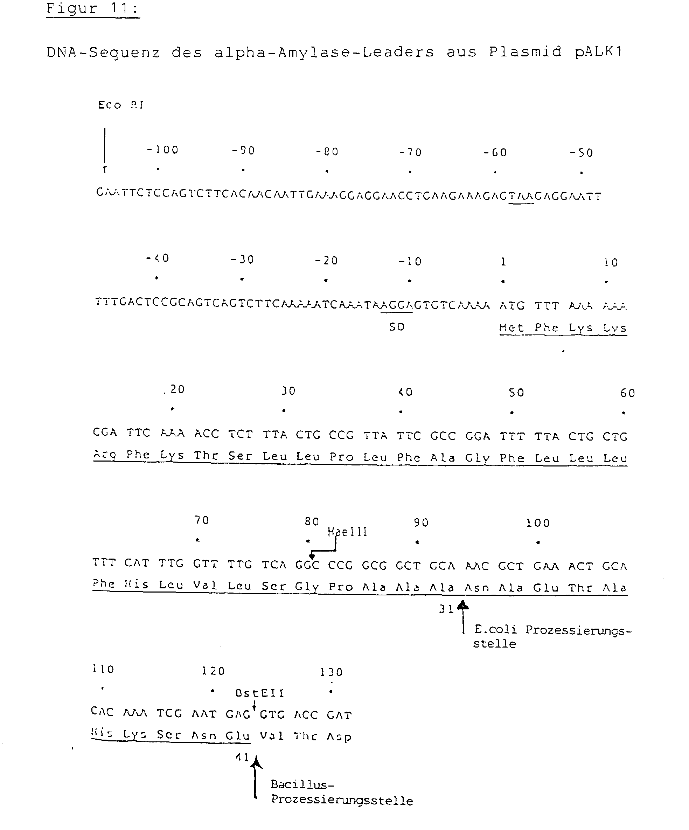

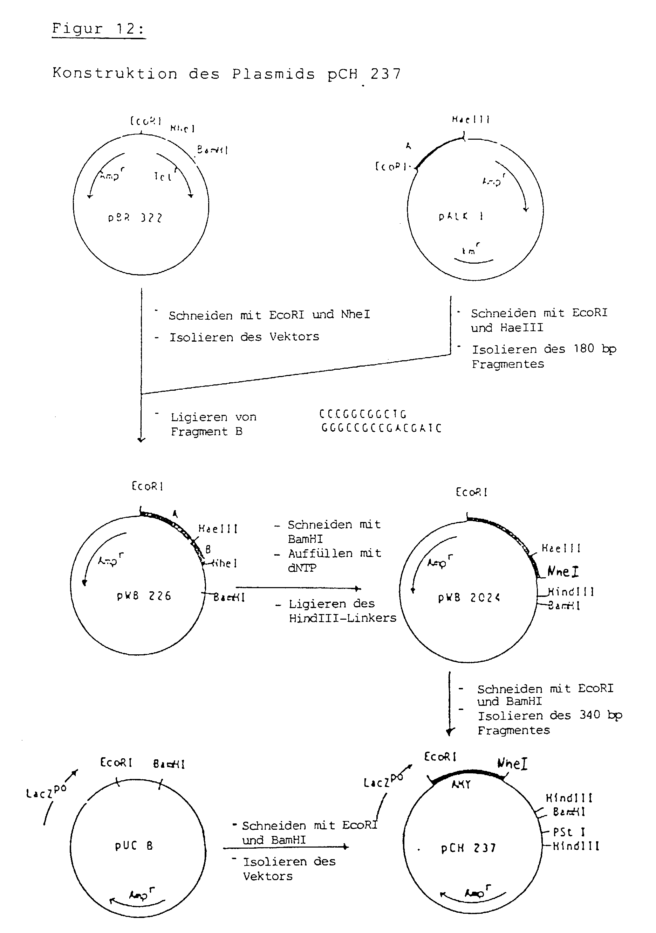

Die alpha-Amylase-Signalsequenz in B. subtilis wurde aus Bacillus subtilis DSM 704 (Deutsche Stammsammlung für Mikroorganismen, Göttingen) durch Klonierung eines partiellen Sau3A-Spaltproduktes der chromosomalen DNA in die BamHI-Stelle von pMK 3 erhalten (M. A. Sullivan et al (1984), Gene 29, 21-26). Einer der Klone, welcher ein 3 Kb DNA-Fragment mit dem alpha-Amylasegen enthielt. wurde durch Deletion von Teilen des alpha-Amylase-Strukturgens modifiziert, um pALK1 zu erhalten (persönliche Mitteilung von Dr. M. A. Courtney; Universität of Rochester, N. Y., mikrobiologische Abteilung). DNA-Sequenzen von pALK1 zeigten eine mögliche Ribosomenbindungsstelle (RBS) und eine Signalsequenz auf einem 230 pb EcoRI-BstEII-Fragment mit weitgehender Homologie zur alpha-Amylase aus B. subtilis 1A289 (vergleiche DNA-Sequenz in Fig. 11 mit M. Yang et al (1983), Nucleic Acid Research, 11, 237-249). Da die Prozessierung der alpha-Amylase-Signalsequenz in E.coli nach der Aminosäure Alanin in Position 31 erfolgt (W. Bruns, nichtveröffentlichte Ergebnisse),führten wir eine Nhel-Restriktionsstelle bei ala 31 ein. Dazu isolierten wir aus pALK1 (von Dr. M. A. Courtney, Rochester, zur Verfügung gestellt) ein 180 bp EcoRI - HaeIII-Fragment, welches die mögliche Shine-Dalgarno-Stelle und einen großen Teil der Signalsequenz von alpha-Amylase enthielt (Fragment A in Fig. 12). Fragment B (s. Fig. 12), ein synthetischer Linker, welcher die Nhel-Stelle am Codon 31 der Amylase-Signalsequenz bildet, wurde zusammen mit dem Fragment A (s. Fig. 12) in pBR 322 ligiert, welches mit EcoRI und Nhel gespalten war (pWB 226). pWB 226 wurde an BamHI restriktiert, und nach Auffüllung mit dNTP wurde ein HindIII-Linker (Biolabs 1002) in den Vektor unter Erhalt von pWB 2024 ligiert.

Der Sekretionsvektor pCH 237 wurde konstruiert, indem die Amylase-Signalsequenz als ECO RI-Bam HI-Fragment aus pWB 2024 hinter den lacZ-Promotor in pUC 8 gebracht wurde (Figur 12). Bei diesem Konstrukt sollte das Leseraster von lacZ' am TAA-Stoppcodon terminiert werden (pos. -58/-56 in der DNA-Sequenz von Fragment A, Fig. 11). Die Reinitiierung der Proteinsynthese auf der gleichen mRNA sollte nach Bindung des Ribosoms an die mögliche Shine-Dalgarno-Stelle von alpha-Amylase ca. 50 Basen stromabwärts vom Stoppcodon in Position -12 erfolgen.

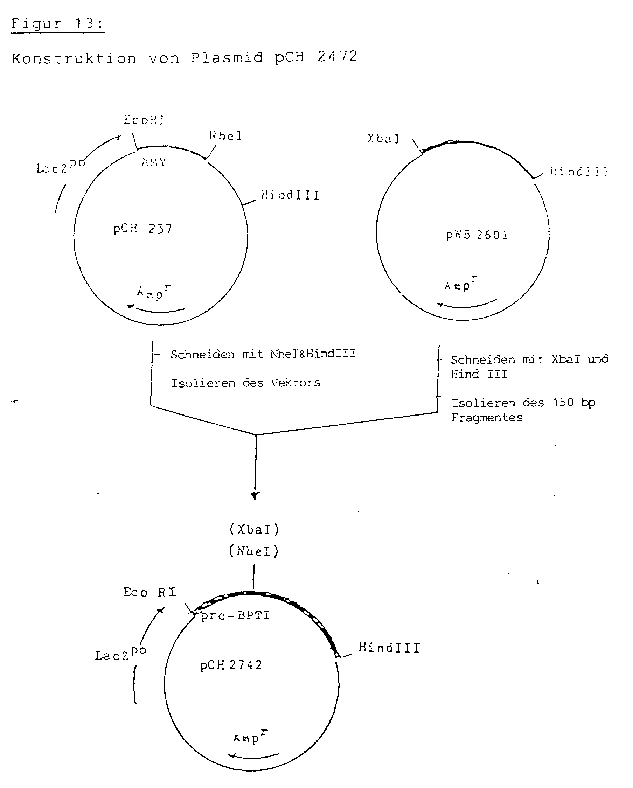

Zur Expression von Val-15-Leu-17-Met-52-Aprotinin wurde das Aprotiningen als Xbal-HindIII-Fragment aus pWB 2601 isoliert und hinter die alpha-Amylase-Signalsequenz in pCH 237, restriktiert mit Nhel und HindIII, integriert, was zu pCH 2742 führt (Fig. 13).

Beispiel 6

Isolierung eines MS-2-Val-15-Leu-17-Glu-39-Glu-52-Aprotinins und/oder eines MS-2-Val-15-Leu-17-Glu-52-Aprotinin-Fusionproteins, welche in E.coli exprimiert wurden

Zur Expression von MS-2-Aprotinin-Fusionsproteinen wurde E.coli C600[pcl 857] mit dem Plasmid pNH 05.1.1 oder pNH 21.1.1 transformiert, in einer Brühe, welche 30 g/l Hefeextrakt, 30 g/l Rindfleischextrakt und 1 g/l $K_2HPO_4$ in entionisiertem Wasser enthielt, gezüchtet. Der pH wurde auf 7,0 eingestellt. 100 ml der Brühe wurden in 1 l Erlenmeyer-Kolben gegeben und 20 min bei 121°C im Autoklaven behandelt. Nach Inkubation mit einer Impfkultur, welche im gleichen Medium 8 h bei 28°C gezüchtet worden war, wurden die Kolben auf einem Rotationsschüttler bei 280 Upm (Durchmesser der Schüttelbewegung: 5 cm) 4 h bei 28°C inkubiert. Zu dieser Zeit betrug die Extinktion der Kultur etwa 4 (gemessen bei 700 nm). Die Temperatur wurde dann auf 42°C erhöht und die Inkubation weitere 3 h fortgesetzt. Zu dieser Zeit enthielten die Zellen bis zu 15 % Fusionsprotein, bezogen auf das gesamte Zellprotein. Das Fusionsprotein konnte durch Standard-Polyacrylamid-Gelelektrophorese unter Verwendung einer 17 %igen Acrylamidkonzentration und Anfärben mit Coomassieblau sichtbar gemacht werden.

Aus der Kulturbrühe wurden wärme-induzierte Zellen durch 15-minütige Zentrifugation bei 5.000 Upm (Beckman JA-10) gesammelt, in 10 ml/g (nach dem Naßgewicht) Puffer A (0,1 M Tris-HCl, pH 7,5, welcher 10 mM EDTA, 5 mM beta-Mercaptoethanol und 5 mM Benzamidin-HCl enthielt) resuspendiert und 30 min bei 30°C mit 0,2 mg/ml Lysozym (ca. 100.000 Einheiten/mg, Fluka AG, Schweiz) unter kräftigem Rühren inkubiert.

Die Suspension wurde dann auf 4°C abgekühlt und zweimal durch eine "French Pressure"-Zelle (Aminco, USA) bei 18.000 psi zum Aufbrechen der Zellmembranen gegeben. Das unlösliche Material wurde durch 30-minütige Zentrifugation bei 10.000 Upm (Beckman JA-10) gewonnen und der Überstand verworfen.

Das Pellet wurde, wie vorstehend beschrieben, zweimal in Puffer A, dem 2 M Harnstoff zugegeben war, resuspendiert und zentrifugiert. Die Überstände wurden wiederum verworfen.

Das Pellet wurde dann in 10 ml/g (Naßgewicht) Puffer B aufgelöst (0,05 M Tris-HCl pH 8,5, welcher 8 M Guanidin-HCl und 10 mM beta-Mercaptoethanol enthielt), die Lösung wurde durch 30-minütige Zentrifugation bei 18.000 Upm (Beckman JA-20) geklärt und 10 ml auf eine Säule (5 x 90 cm), die mit Sephacryl S-300 (Pharmacia AB, Schweden) gefüllt und in Puffer C (0,05 M Tris-HCl pH 8,5, welcher 6 M Harnstoff und 10 mM beta-Mercaptoethanol enthielt) equilibriert war, aufgegeben. Fraktionen von 10 ml wurden gesammelt und der Peak, der das Fusionsprotein enthielt, mittels SDS-PAGE unter Reduktionsbedingungen identifiziert. Die Peakfraktionen wurden vereinigt und ausgiebig gegen Wasser dialysiert. Unter diesen Bedingungen fiel das Fusionsprotein aus und wurde durch 30-minütige Zentrifugation bei 10.000 Upm (Beckman JA-10) gesammelt. Die Fusionsproteinpellets wurden bei -70°C gelagert.

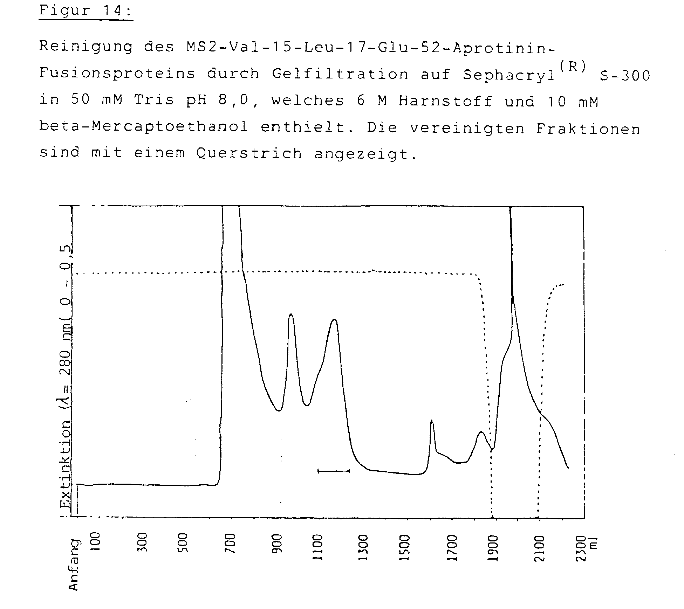

Fig. 14 zeigt eine typische Trennung. Fraktionen, die das Fusionsprotein enthalten, sind durch einen Balken gekennzeichnet.

### Beispiel 7

#### Herstellung von bioaktivem Val-15-Leu-17-Glu-52-Aprotinin aus gereinigtem MS-2-Fusionsprotein

Das Fusionsprotein (hergestellt nach Beispiel 6) wurde in ca. 5 ml/100 mg (Naßgewicht) 70 %iger Ameisensäure aufgelöst und mit Cyanogenbromid (molares Verhältnis Methionin: CNBr = 1:250) 18 h unter Stickstoff bei Raumtemperatur behandelt (Witkop et al (1968), Science 162, 318-326). Das Spaltgemisch wurde dann auf auf die 10- bis 20-fache Menge mit Wasser verdünnt; Ameisensäure und restliches CNBr wurden unter reduziertem Druck entfernt.

Die konzentrierte Lösung wurde mit 5 M NaOH auf pH 7,5 titriert und fester Harnstoff bis zu einer Endkonzentration von 8 molar zugegeben. Nach Zugabe von 25 mM beta-Mercaptoethanol und 2-stündiger Inkubation bei 37°C unter Stickstoff wurde die Lösung über Nacht gegen 20 Volumen Puffer D (0,05 M Na-acetat pH 5,2, welches 6 M Harnstoff und 10 mM beta-Mercaptoethanol enthielt) bei 8°C dialysiert.

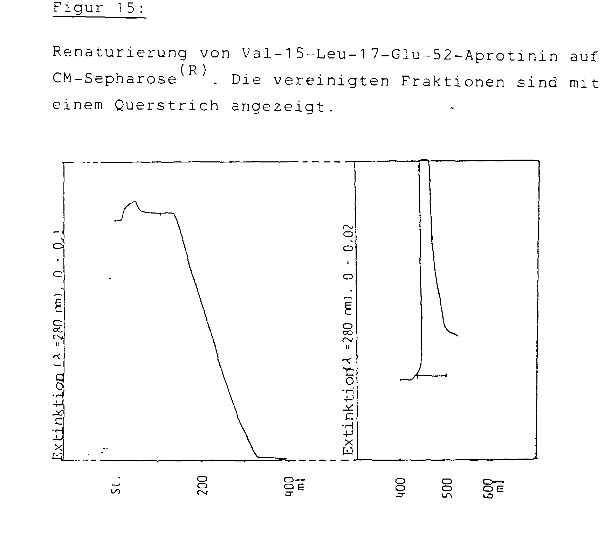

Um Val-15-Leu-17-Glu-52-Aprotinin zu renaturieren, wurde die Lösung auf eine Säule (2,5 x 5 cm), welche mit CM-Sepharose fast flow (Pharmacia AB, Schweden) gefüllt und in Puffer D equilibriert war, gegeben. Die Säule wurde mit Puffer D (ca. 8 bis 10 Säulenvolumen) gewaschen und mit einem linearen Gradienten aus 150 ml Puffer D und 150 ml Puffer E (0,05 M Na-acetat, pH 5,2, welcher 2 mM beta-Mercaptoethanol enthielt) entwickelt, worauf kurz mit 0,05 M Na-acetat pH 5,2 (ca. 2 bis 3 Säulenvolumen) gewaschen wurde. Schließlich wurde renaturiertes Val-15-Leu-17-Glu-52-Aprotinin mit 0,05 M Na-acetat pH 5,2, welches 0,5 M NaCl enthielt, eluiert (s. Fig. 15).

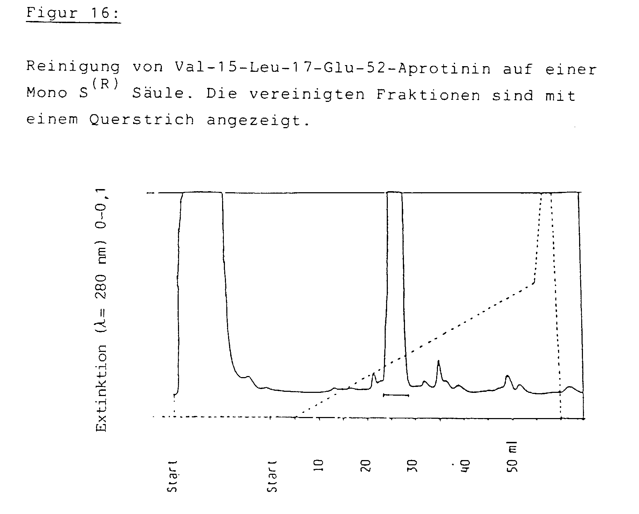

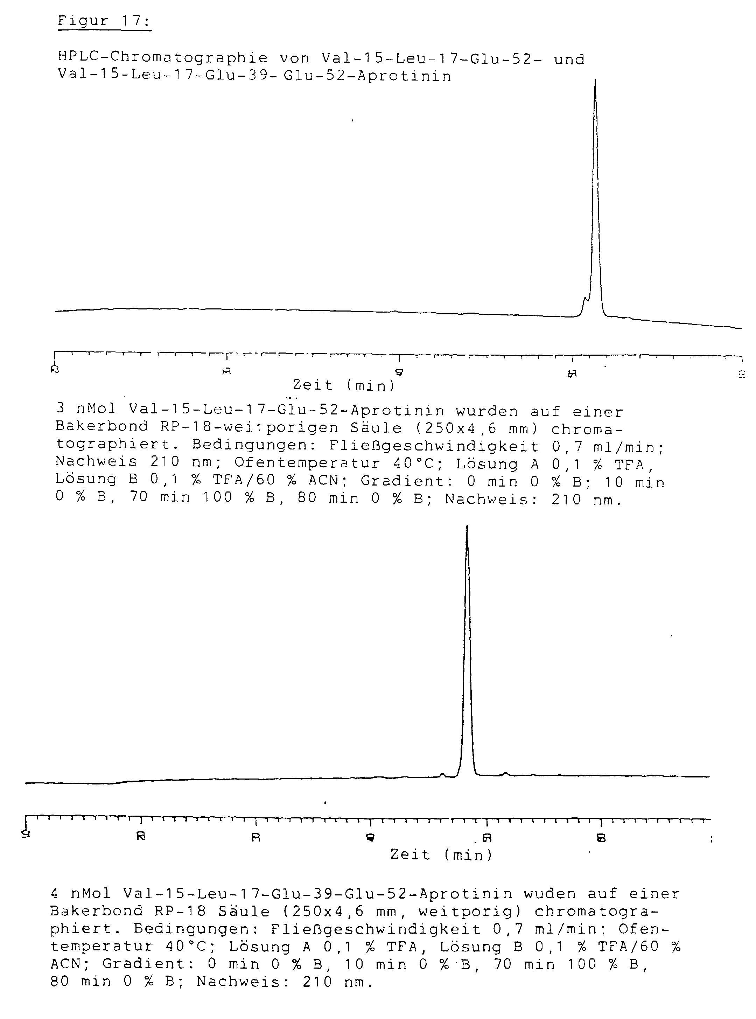

Die Peakfraktionen des NaCl-Eluats (identifiziert mit SDS-PAGE) wurden gesammelt und ausgiebig gegen 20 mM Hepes pH 6,5 dialysiert und im folgenden auf einer Mono-S-Säule (1 ml), die mit dem gleichen Puffer equilibriert war (FPLC, Pharmacia, Schweden),gereinigt. Das Protein wurde an die Säule gebunden und mit einem linearen Gradienten von Null bis 300 mM NaCl eluiert (s. Fig. 16). Die Peakfraktionen, welche die Aprotininvariante enthielten, wurden gesammelt, ausgiebig gegen 0,1 M Ammoniumbicarbonat dialysiert und in geeigneten aliquoten Teilen lyophilisiert.

Es wurden gewöhnlich 0,5 bis 1,5 mg gereinigtes Val-15-Leu-17-Glu-52-Aprotinin pro Liter Kulturbrühe gewonnen.

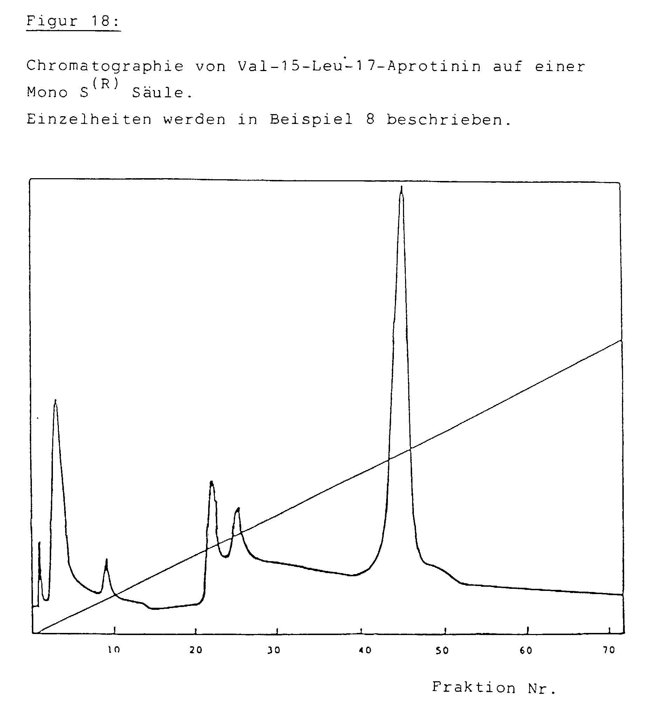

Das gereinigte Protein wies die erwarteten Eigenschaften im Hinblick auf Aminosäurezusammensetzung, lineare Sequenz (Position 1 - 25), Molekulargewicht und Verhalten auf Umkehrphasen-HPLC (Fig. 17) auf (s. Beispiel 9).

### Beispiel 8

#### Isolierung von Val-15-Leu-17-Aprotinin, exprimiert in E.coli RRI M15 pCH 2742

Mit pWB 2742 transformierte E.coli RRI M15 wurden in Übernacht-Kulturen gezüchtet. Das Medium enthielt 3 % Rinderextrakt (Gibco), 1,5 % Hefeextrakt (Gibco), 0,5 % $K_2HPO_4$ und 4 % Morpholino-ethan-sulfonsäure, aufgelöst in destilliertem Wasser. Der pH-Wert wurde auf 7,0 eingestellt. 100 ml des Mediums wurden in 1 l Erlenmeyer-Kolben gegeben und 20 min bei 121°C im Autoklaven behandelt. Nach dem Abkühlen wurde Ampicillin, welches in destilliertem Wasser aufgelöst und durch Filtration sterilisiert worden war, bis zu einer Endkonzentration von 50 μg/l zugegeben. Zur Herstellung von Val-15-Leu-17-Aprotinin wurden die Kolben mit einer über Nacht in dem gleichen Medium bei 28°C gezüchteten Kultur inokuliert. Die Kolben wurden 3 h auf einem Rotationsschüttler bei 280 Upm und 28°C inkubiert, bis die bei 550 nm gemessene Extinktion ca. 1 betrug. Dann erfolgte Induktion des lac-Promotors durch Zugabe von 1 mM Isopropylthiogalactosid (Sigma) zu den Kulturen. Die Fermentation wurde 20 h lang fortgesetzt und die Zellen dann durch Zentrifugation bei 8.000 Upm (10 min 4°C in Kontron Centrikon H-401 mit Rotor A 6.14) geerntet.

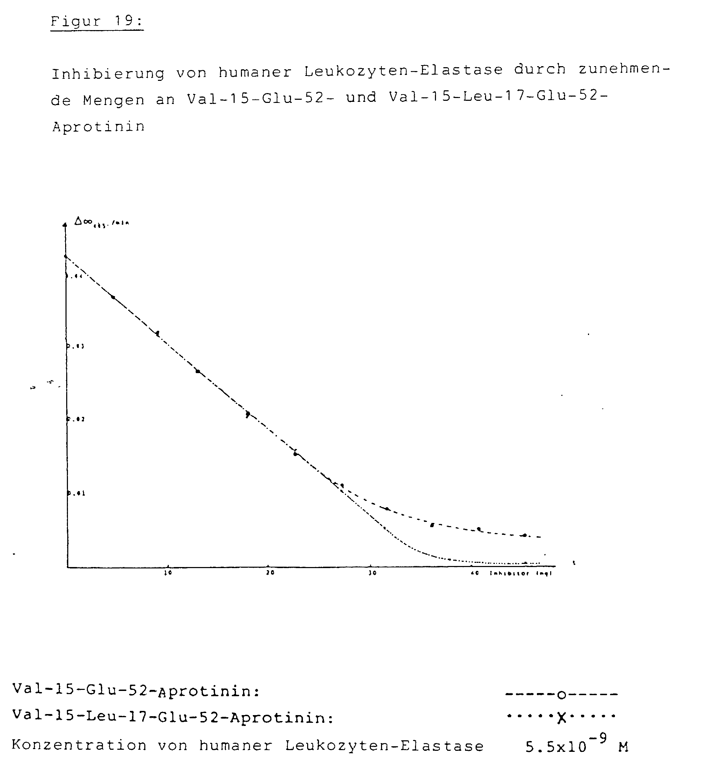

Die Zellen wurden in 500 ml 0,01 M Tris-Puffer pH 8,0 homogenisiert und unter Kühlung mit zerkleinertem Eis durch Ultrabeschallung bei 400 W aufgebrochen, bis mehr als 95 % sämtlicher Zellen zerstört waren. 30 ml Perchlorsäure (72 %ig) wurde zu dieser Suspension (700 ml) unter Rühren zugegeben. Nach 30 min wurde der Überstand durch Zentrifugation (20 min/6.000 Upm) gewonnen. Er wurde durch Zugabe von gesättigter Tris-Basenlösung neutralisiert und über 50 ml Gelbett-Anti-Aprotinin-Antikörper (Immunisation erfolgte gegen Aprotinin in Kaninchen), welche an Sepharose CL 4B nach der BrCN-Methode immobilisiert waren, gegeben. Im Durchlauf wurde kein aktives Material gefunden. Das Gel wurde nacheinander mit 0,2 M Tris-Puffer pH 8,0 und Wasser gewaschen, wobei keine Aktivität desorbiert wurde. Die Aktivität konnte durch Elution mit 0,2 M Essigsäure, eingestellt mit HCl auf pH 1,9, desorbiert werden. Dieses Eluat wurde lyophilisiert und in 5 ml 0,02 M Hepes-Puffer pH 6,0 aufgelöst. Eine weitere Reinigung wurde durch FPLC auf Mono S[(R)] (Pharmacia, Schweden) unter Verwendung eines ansteigenden Gradienten von NaCl (0 bis 0,5 M) erzielt. Die Aktivität lag in den Fraktionen 41-47 vor (s. Fig. 18), die vereinigt, dialysiert und lyophilisiert

EP 0 307 592 B1

wurden.

Beispiel 9

Chemische Proteincharakterisierung von Val-15-Leu-17-Aprotinin-Varianten

Eine vollständige Aminosäureanalyse wurde durchgeführt, wie dies in Materialien und Methoden beschrieben ist. Die erzielten Ergebnisse, welche die Aminosäurezusammensetzung von

Val-15-Leu-17-Glu-52-Aprotinin,
Val-15-Leu-17-Thr-52-Aprotinin,
Leu-15-Leu-17-Glu-52-Aprotinin,
Val-15-Leu-17-Glu-39-Glu 52-Aprotinin und
Val-15-Leu-17-Met-52-Aprotinin

betraf, waren folgende:

| Aminosäure | 1* | 2* | 3* | 4* | 5* |
|---|---|---|---|---|---|
| Asp | 5,09(5) | 5,18(5) | 5,15(5) | 4,95(5) | 5,13(5) |
| Thr | 3,03(3) | 3,89(4) | 2,83(3) | 2,81(3) | 2,87(3) |
| Ser | 1,19(1) | 1,13(1) | 1,02(1) | 1,15(1) | 1,01(1) |
| Glu | 4,43(4) | 3,31(3) | 4,44(4) | 5,57(5) | 3,20(3) |
| Ala | 6,00(6) | 6,00(6) | 6,00(6) | 5,84(6) | 6,31(6) |
| Gly | 6,28(6) | 5,86(6) | 5,84(6) | 5,92(6) | 6,02(6) |
| Val | 2,13(2) | 1,97(2) | 0,98(1) | 2,00(2) | 1,87(2) |
| Met | ----(-) | ----(-) | ----(-) | ----(-) | 0,88(1) |
| Ile | 1,45(2) | 1,35(2) | 1,39(2) | 1,42(2) | 1,28(2) |
| Leu | 2,97(3) | 2,87(2) | 3,87(4) | 2,89(3) | 2,90(3) |
| Tyr | 3,73(4) | 3,64(4) | 3,73(4) | 3,09(4) | 3,58(4) |
| Phe | 3,83(4) | 3,83(4) | 3,83(4) | 3,69(4) | 3,83(4) |
| Lys | 2,84(3) | 2,79(3) | 2,92(3) | 2,85(3) | 2,80(3) |
| Arg | 4,82(5) | 4,76(5) | 4,95(5) | 3,79(4) | 4,77(5) |

1* Val-15-Leu-17-Glu-52-Aprotinin
2* Val-15-Leu-17-Thr-52-Aprotinin
3* Leu-15-Leu-17-Glu-52-Aprotinin
4* Val-15-Leu-17-Glu-39-Glu-52-Aprotinin
5* Val-15-Leu-17-Met-52-Aprotinin

Cys und Pro wurden nicht bestimmt.

Die N-terminale Aminosäuresequenz wurde bestimmt, wie dies in Materialien und Methoden angegeben ist. Die Aminosäuresequenz, wie sie nach 25 Zyklen erhalten wurde, war folgende:

Val-15-Leu-17-Glu-52-Aprotinin:

1
Arg-Pro-Asp-Phe-Cys-Leu-Glu-Pro-Pro-Tyr-Thr-Gly-Pro-Cys-Val-

17  18                          25
-Ala-Leu-Ile-Ile-Arg-Tyr-Phe-Tyr-Asn-Ala-

18

```
Val-15-Leu-17-Thr-52-Aprotinin
 1
Arg-Pro-Asp-Phe-Cys-Leu-Glu-Pro-Pro-Tyr-Thr-Gly-Pro-Cys-Val-
        17  18                               25
Ala-Leu-Ile-Ile-Arg-Tyr-Phe-Tyr-Asn-Ala-
```

```
Leu-15-Leu-17-Glu-52-Aprotinin
   1
Arg-Pro-Asp-Phe-Cys-Leu-Glu-Pro-Pro-Tyr-Thr-Gly-Pro-Cys-Leu-
        17  18                               25
-Ala-Leu-Ile-Ile-Arg-Tyr-Phe-Tyr-Asn-Ala-
```

```
Val-15-Leu-17-Glu-39-Glu-52-Aprotinin
   1
Arg-Pro-Asp-Phe-Cys-Leu-Glu-Pro-Pro-Tyr-Thr-Gly-Pro-Cys-Val-
        17  18                               25
-Ala-Leu-Ile-Ile-Arg-Tyr-Phe-Tyr-Asn-Ala-
```

```
Val-15-Leu-17-Met-52-Aprotinin
   1
Arg-Pro-Asp-Phe-Cys-Leu-Glu-Pro-Pro-Tyr-Thr-Gly-Pro-Cys-Val-
        17  18                               25
-Ala-Leu-Ile-Ile-Arg-Tyr-Phe-Tyr-Asn-Ala-
```

Ca. 30 % des durch Fermentation in E.coli RR1 Δ M15 pCH 2742 erhaltenen Val-15-Leu-17-Met-52-Aprotinins enthielten einen zusätzlichen Ala-Rest am N-Terminus. Dies kann verhindert werden, indem man in der alpha-Amylase-Signalsequenz (s. Fig. 11) Ala-30 durch Gln ersetzt.

Beispiel 10

Bestimmung der kinetischen Konstanten von Val-15-Leu-17-Aprotininvarianten

Die $K_i$-Werte wurden bestimmt, wie dies in Materialien und Methoden angegeben ist. Die Inhibierung humaner Leukozyten-Elastase (HLE) durch zunehmende Mengen an Val-15-Glu-52- und Val-15-Leu-17-Glu-52-Aprotinin ist in Fig. 18 wiedergegeben. Typische $K_i$-Werte für die Inhibierung von humaner Leukozyten-Elastase, humaner pankreatischer Elastase I und humanem Cathepsin G, wie sie mit Aprotininvarianten erhalten wurden, sind folgende:

| | $K_i$ (M) | | |
| --- | --- | --- | --- |
| | Humane Leukozyten-Elastase | Humane pankreatische Elastase I | Humanes Cathepsin G |
| Val-15-Glu-52 | $1,5 \times 10^{-10}$ | – | – |
| Val-15-Leu-17-Glu-52 | $5-6 \times 10^{-11}$ | $>10^{-6}$ | – |
| Val-15-Leu-17-Glu -39-Thr-52 | $2 \times 10^{-10}$ | $5 \times 10^{-9}$ | – |
| Val-15-Leu-17-Met-52 | $6 \times 10^{-11}$ | $>10^{-6}$ | – |
| Val-15-Leu-17-Thr-52 | $7 \times 10^{-11}$ | $>10^{-6}$ | – |
| Leu-15-Leu-17-Glu-52 | $1 \times 10^{-10}$ | $>10^{-7}$ | $2 \times 10^{-8}$ |
| Leu-15-Leu-17-Glu-39-Glu-52 | $2 \times 10^{-9}$ | $1 \times 10^{-9}$ | $1 \times 10^{-9}$ |

**Patentansprüche**

1. Peptide mit der Sequenz von pankreatischem Rinder-Trypsininhibitor (Aprotinin, BPTI),

mit einem Austauch der natürlichen Aminosäure Arg in der Position 17 durch Leu, Ile oder Ala
wobei eine oder mehrere Aminosäuren in der Position 15, 16; 18, 34, 39 and 52 durch eine der folgenden aminosäuren ausgetauscht ist bzw. sind, und zwar
in Position 15 durch die Aminosäure Leu, Ile, Val, Phe, Tyr, Trp, Met, Ala, Thr, Ser, Gln, Asn oder Arg,
in Position 16 durch die Aminosäure Val, Met, Thr, Ser, Gln, Asn, Gly oder Arg,
in Position 18 durch die Aminosäure Leu, Val, Phe, Met, Thr, Glu oder Gly,
in Position 34 durch die Aminosäure Leu, Ile, Phe, Tyr, Trp, Ala oder Thr
in Position 39 durch die Aminosäüre Leu, Ile, Val, Phe, Tyr, Trp, Met, Ala, Thr, Ser, Glu, Gln, Asn, Gly, Lys, Asp oder Pro
und in Position 52 durch die Aminosäure Leu, Ile, Val, Thr, Ser, Glu, Gln, Asp, Lys oder Arg,

ausgenommen Val 15-Ser-16 Ile-17 Aprotinin.

2. Peptide nach Anspruch 1, die aufweisen:

in Position 15 die Aminosäure Val, Leu oder Ile,
in Position 39 die Aminosäure Glu, Asp, Asn, Thr, Val, Leu, Ile oder Gln und in
Position 52 die Aminosäure Thr oder Glu.

3. Peptide nach Anspruch 1, welche aufweisen:

in Position 15 die Aminosäure Val, Leu oder Ile,
in Position 39 die Aminosäure Glu oder in

Position 52 die Aminosäure Thr oder Glu.

**4.** Peptide nach Anspruch 1, welche aufweisen:

in Position 15 die Aminosäure Val, Leu oder Ile,
in Position 39 die Aminosäure Glu oder in
Position 52 die Aminosäure Thr oder Glu.

**5.** Peptide nach Anspruch 1, welche aufweisen:

in Position 15 die Aminosäure Val oder Leu,
in Position 39 die Aminosäure Glu oder in
Position 52 die Aminosäure Thr oder Glu.

**6.** Peptide nach Anspruch 1:

Val-15-Leu-17-
Val-15-Leu-17-Thr-52-
Val-15-Leu-17-Thr-52-
Val-15-Leu-17-Glu-39-
Val-15-Leu-17-Glu-39-Glu-52-
Val-15-Leu-17-Glu-39-Thr-52-
Leu-15-Leu-17-
Leu-15-Leu-17-Glu-52-
Leu-15-Leu-17-Thr-52-
Leu-15-Leu-17-Glu-39-
Leu-15-Leu-17-Glu-39-Glu-52-
Leu-15-Leu-17-Glu-39-Thr-52-Aprotinin.

**7.** Peptide nach einem oder mehreren der Ansprüche 1 bis 6, welche eine Aminosäure oder eine Peptidsequenz aufweisen, die der Aminosäure Arg in Position 1 vorausgeht oder auf die Aminosäure Ala in Position 58 folgt.

**8.** Peptide nach Ansprüchen 1 bis 7, welche zusätzlich Methionin in Position -1 und/oder ein Leaderpeptid aufweisen.

**9.** Peptide gemäß Anspruch 5, welche an einem oder an beiden Enden des Moleküle durch eine oder mehrere Aminosäuren verkürzt sind.

**10.** Peptide nach einem oder mehreren der Ansprüche 1 bis 9, hergestellt durch rekombinante DNA-Technologie.

**11.** DNA, welche das Peptid nach einem oder mehreren der Ansprüche 1 bis 10 kodiert.

**12.** DNA nach Anspruch 11, welche im weiteren stromaufwärts ein Codon für Methionin oder eine DNA, welche für ein Leaderpeptid kodiert oder stromabwärtd Codons für ein oder mehrere zusätzliche Aminosäuren aufweist.

**13.** Expressionsvektor, welcher die DNA nach Anspruch 11 oder 12 umfaßt.

**14.** Wirtsorganismus, welcher mit dem Expressionsvektor nach Anspruch 13 transformiert ist, wobei der wirtsorganismus Gram-negative und Grampositive Bakterien, Hefen oder Fadenpilze darstellen kann.

**15.** Verwendung des Wirtsorganimus nach Anspruch 14 zur Herstellung des Peptids nach einem oder mehreren der Ansprüche 1 bis 9.

**16.** Verfahren zur Herstellung des Peptids nach einem oder mehreren der Ansprüche 1 bis 9, gekennzeichnet durch die Schritte:

a) Züchten eines Wirtsorganismus unter geeigneten Bedingungen,
b) Gewinnung des Peptids aus der Kultur,
c) Reinigen des Peptids, dadurch gekennzeichnet, daß der Wirtsorganismus mit dem Expressionsvektor nach Anspruch 13 transformiert ist.

**17.** Verwendung des Peptids nach einem oder mehreren der Ansprüche 1 bis 9 zur Herstellung von Arzneimitteln.

**18.** Pharmazeutische Zubereitung, dadurch gekennzeichnet, daß sie Peptide nach einem oder mehreren der Ansprüche 1 bis 9 umfaßt.

**19.** E. Coli RRI ΔM15 pES 44.1.1 (DSM 4157),
E. Coli RRI ΔM15 pES 45.1.3 (DMS 4158),
E. Coli RRI ΔM15 pCH 2742 (DMS 4159).

**Claims**

**1.** Peptides having the sequence of bovine pancreatic trypsin inhibitor (aprotinin, BPTI),

with a replacement of the natural amino acid Arg in position 17 by Leu, Ile or Ala,
with one or more amino acids in positions 15, 16, 18, 34, 39 and 52 being replaced by one of the following amino acids, i.e.
in position 15, by the amino acid Leu, Ile, Val, Phe, Tyr, Trp, Met, Ala, Thr, Ser, Gln, Asn or Arg,
in position 16, by the amino acid Val, Met, Thr, Ser, Gln, Asn, Gly or Arg,
in position 18, by the amino acid Leu, Val, Phe, Met, Thr, Glu or Gly,
in position 34, by the amino acid Leu, Ile, Phe, Tyr, Trp, Ala or Thr,
in position 39, by the amino acid Leu, Ile, Val, Phe, Tyr, Trp, Met, Ala, Thr, Ser, Glu, Gln, Asn, Gly, Lys, Asp or Pro,
and in position 52, by the amino acid Leu, Ile, Val, Thr, Ser, Glu, Gln, Asp, Lys or Arg,
with the exception of Val-15-Ser-16-Ile-17 aprotinin.

**2.** Peptides according to Claim 1 which exhibit:

in position 15, the amino acid Val, Leu or Ile,
in position 39, the amino acid Glu, Asp, Asn, Thr, Val, Leu, Ile or Gln,
and in
position 52, the amino acid Thr or Glu.

**3.** Peptides according to Claim 1 which exhibit:

in position 15, the amino acid Val, Leu or Ile,
in position 39, the amino acid Glu, or in
position 52, the amino acid Thr or Glu.

**4.** Peptides according to Claim 1 which exhibit:

in position 15, the amino acid Val, Leu or Ile,
in position 39, the amino acid Glu, or in
position 52, the amino acid Thr or Glu.

**5.** Peptides according to Claim 1 which exhibit:

in position 15, the amino acid Val or Leu,
in position 39, the amino acid Glu, or in
position 52, the amino acid Thr or Glu.

**6.** Peptides according to Claim 1:

Val-15-Leu-17-
Val-15-Leu-17-Thr-52-
Val-15-Leu-17-Thr-52-

Val-15-Leu-17-Glu-39-
Val-15-Leu-17-Glu-39-Glu-52-
Val-15-Leu-17-Glu-39-Thr-52-
Leu-15-Leu-17-
Leu-15-Leu-17-Glu-52-
Leu-15-Leu-17-Thr-52-
Leu-15-Leu-17-Glu-39-
Leu-15-Leu-17-Glu-39-Glu-52-
Leu-15-Leu-17-Glu-39-Thr-52-aprotinin.

7. Peptides according to one or more of Claims 1 to 6 which exhibit an amino acid or a peptide sequence which precedes the amino acid Arg in position 1 or succeeds the amino acid Ala in position 58.

8. Peptides according to Claims 1 to 7 which additionally exhibit methionine in position -1 and/or a leader peptide.

9. Peptides according to Claim 6 which are truncated by one or more amino acids at one or both ends of the molecule.

10. Peptides according to one or more of Claims 1 to 9 which are prepared by recombinant DNA technology.

11. DNA which encodes the peptide according to one or more of Claims 1 to 10.

12. DNA according to Claim 11 which additionally exhibits upstream a codon for methionine or a DNA which encodes a leader peptide or downstream codons for one or more additional amino acids.

13. Expression vector which encompasses the DNA according to Claim 11 or 12.

14. Host organism which is transformed with the expression vector according to Claim 13, with it being possible for the host organism to be Gram-negative or Gram-positive bacteria, yeasts or filamentous fungi.

15. Use of the host organism according to Claim 14 for preparing the peptide according to one or more of Claims 1 to 9.

16. Process for preparing the peptide according to one or more of Claims 1 to 9, characterized by the steps:

a) culturing a host organism under suitable conditions,
b) isolating the peptide from the culture,
c) purifying the peptide, characterized in that the host organism is transformed with the expression vector according to Claim 13.

17. Use of the peptide according to one or more of Claims 1 to 9 for preparing medicaments.

18. Pharmaceutical preparation, characterized in that it comprises peptides according to one or more of Claims 1 to 9.

19. E. Coli RRI ΔM15 pES 44.1.1 (DSM 4157),
E. Coli RRI ΔM15 pES 45.1.3 (DMS 4158),
E. Coli RRI ΔM15 pCH 2742 (DMS 4159).

**Revendications**

1. Peptides comprenant la séquence de l'inhibiteur de la trypsine pancréatique bovine (aprotinine, BPTI),

dans laquelle on a échangé l'aminoacide naturel Arg en position 17 par Leu, Ile ou Ala,
dans lesquels un ou plusieurs aminoacides dans les positions 15, 16, 18, 34, 39 et 52 a ou ont été échangés par un des aminoacides ci-après, plus précisément:
en position 15, par l'aminoacide Leu, Ile, Val, Phe, Tyr, Trp, Met, Ala, Thr, Ser, Gln, Asn ou Arg,
en position 16, par l'aminoacide Val, Met, Thr, Ser, Gln, Asn, Gly ou Arg,
en position 18, par l'aminoacide Leu, Val, Phe, Met, Thr, Glu ou Gly,
en position 34, par l'aminoacide Leu, Ile, Phe, Tyr, Trp, Ala ou Thr,

en position 39, par l'aminoacide Leu, Ile, Val, Phe, Tyr, Trp, Met, Ala, Thr, Ser, Glu, Gln, Asn, Gly, Lys, Asp ou Pro, et
en position 52, par l'aminoacide Leu, Ile, Val, Thr, Ser, Glu, Gln, Asp, Lys ou Arg,
à l'exception de la Val-15-Ser-16-Ile-17- aprotinine.

2. Peptides selon la revendication 1, qui présentent:

en position 15, l'aminoacide Val, Leu ou Ile,
en position 39, l'aminoacide Glu, Asp, Asn, Thr, Val, Leu, Ile ou Gln, et
en position 52, l'aminoacide Thr ou Glu.

3. Peptides selon la revendication 1, qui présentent:

en position 15, l'aminoacide Val, Leu ou Ile,
en position 39, l'aminoacide Glu ou
en position 52, l'aminoacide Thr ou Glu.

4. Peptides selon la revendication 1, qui présentent:

en position 15, l'aminoacide Val, Leu ou Ile,
en position 39, l'aminoacide Glu ou
en position 52, l'aminoacide Thr ou Glu.

5. Peptides selon la revendication 1, qui présentent:

en position 15, l'aminoacide Val ou Leu,
en position 39, l'aminoacide Glu ou
en position 52, l'aminoacide Thr ou Glu.

6. Peptides selon la revendication 1:

Val-15-Leu-17-
Val-15-Leu-17-thr -52-
Val-15-Leu-17-Thr-52-
Val-15-Leu-17-Glu-39-
Val-15-Leu-17-Glu-39-Glu-52-
Val-15-Leu-17-Glu-39-Thr-52-
Leu-15-Leu-17-
Leu-15-Leu-17-Glu-52-
Leu-15-Leu-17-Thr-52-
Leu-15-Leu-17-Glu-39-
Leu-15-Leu-17-Glu-39-Glu-52-
Leu-15-Leu-17-Glu-39-Thr-52-aprotinine.

7. Peptides selon une ou plusieurs des revendications 1 à 6, qui présentent un aminoacide ou une séquence peptidique qui précède l'aminoacide Arg en position 1 ou qui suit l'aminoacide Ala en position 58.

8. Peptides selon une ou plusieurs des revendications 1 à 7, qui présentent en outre de la méthionine en position 1 et/ou un peptide leader.

9. Peptides selon la revendication 6, qui ont été raccourcis d'un ou de plusieurs aminoacides à une extrémité ou aux deux extrémités de la molécule.

10. Peptides selon une ou plusieurs des revendications 1 à 9, préparés par la technologie de l'ADN recombinant.

11. ADN qui code le peptide selon une ou plusieurs des revendications 1 à 10.

12. ADN selon la revendication 11, qui présente en outre en amont un codon pour la méthionine ou un ADN qui code pour un peptide leader ou en aval des codons pour un ou plusieurs aminoacides supplémentaires.

13. Vecteur d'expression qui comprend l'ADN selon la revendication 11 ou 12.

14. Organisme hôte qui a été transformé avec le vecteur d'expression selon la revendication 13, l'organisme hôte pouvant représenter des bactéries Gram-négatif et Gram-positif, des levures ou des champignons filamenteux.

15. Utilisation de l'organisme hôte selon la revendication 14 pour la préparation du peptide selon une ou plusieurs des revendications 1 à 9.

16. Procédé pour la préparation du peptide selon une ou plusieurs des revendications 1 à 9, caractérisé par les étapes consistant à:

   a) cultiver un organisme hôte dans des conditions appropriées,
   b) récupérer le peptide de la culture,
   c) purifier le peptide, caractérisé en ce que l'organisme hôte a été transformé avec le vecteur d'expression selon la revendication 13.

17. Utilisation du peptide selon une ou plusieurs des revendications 1 à 9 pour la préparation de médicaments.

18. Formulation pharmaceutique caractérisée en ce qu'elle comprend des peptides selon une ou plusieurs des revendications 1 à 9.

19.    E. Coli RRI ΔM15pBS44.1.1 (DSM 4157)
   E. Coli RRI ΔM15pES45.1.3 (DSM 4158)
   E. Coli RRI ΔM15pCH2742 (DSM 4159).

Figur 1:

Konstrukt des synthetischen Val-15-Glu-52-Aprotinin-Mastergens

Figur 2:

DNA-Sequenzen von 15 Oligonukleotid-Fragmenten, wie sie
für die Konstruktion des synthetischen Val-15-Glu-52-
Aprotiningens verwendet wurden


Fra   1    AATTCATGCGTCCGGACTTCTGCCTCGAGC

Fra   2    CAGAAGTCCGGACGCATG

Fra   3    CGCCGTACACTGGGCCCTGCGTTGCT

Fra   4    CCCAGTGTACGGCGGCTCGAGG

Fra   5    CGTATCATCCGTTACTTC

Fra   6    ATGATACGAGCAACGCAGGG

Fra   7    TACAATGCAAAGGCAGGCCTGTGTCAGACC

Fra   8    CCTGCCTTTGCATTGTAGAAGTAACGG

Fra   9    TTCGTATACGGCGGTTGCCGTGCTAAGCGT

Fra  10    AACCGCCGTATACGAAGGTCTGACACAGG

Fra  11    AACAACTTCAAATCCGCGGAAGACTGCGAA

Fra  12    ATTTGAAGTTGTTACGCTTAGCACGGC

Fra  13    CGTACTTGCGGTGGTGCTTAGTAAAGCTTG

Fra  14    CACCGCAAGTACGTTCGCAGTCTTCCGCGG

Fra  16    GATCCAAGCTTTACTAAGCAC

Figur 3:

DNA-Sequenz des synthetischen Val-15-GLu-52-Aprotinin-
Mastergens in Plasmid pRK 54.1.1

```
                                  X                           A
                                  h                           p
                                  o                           a
                                  l                           l
            ArgProAspPheCysLeuGluProProTyrThrGlyProCys -
         AATTCATGCGTCCGGACTTCTGCCTCGAGCCGCCGTACACTGGGCCCTGC
       1 ---------+---------+---------+---------+---------+ 50
         GTACGCAGGCCTGAAGACGGAGCTCGGCGGCATGTGACCCGGGACG


                                                      S
                                                      t
                                                      u
                                                      l
            ValAlaArgIleIleArgTyrPheTyrAsnAlaLysAlaGlyLeuCysGln -
         GTTGCTCGTATCATCCGTTACTTCTACAATGCAAAGGCAGGCCTGTGTCA
      51 ---------+---------+---------+---------+---------+ 100
         CAACGAGCATAGTAGGCAATGAAGATGTTACGTTTCCGTCCGGACACAGT


            ThrPheValTyrGlyGlyCysArgAlaLysArgAsnAsnPheLysSerAla -
         GACCTTCGTATACGGCGGTTGCCGTGCTAAGCGTAACAACTTCAAATCCG
     101 ---------+---------+---------+---------+---------+ 150
         CTGGAAGCATATGCCGCCAACGGCACGATTCGCATTGTTGAAGTTTAGGC


         S                                            H
         s                                            i
         s                                            n
         t                                            d
         2                                            3
            GluAspCysGluArgThrCysGlyGlyAlaEnd
         CGGAAGACTGCGAACGTACTTGCGGTGGTGCTTAGTAAAGCTTG
     151 ---------+---------+---------+---------+---------
         GCCTTCTGACGCTTGCATGAACGCCACCACGAATCATTTCGAACCTAG
```

Figur 4:

Rekombinantes Plasmid pRK 54.1.1

Figur 5:

DNA-Sequenz von Val-15-Leu-17-Glu-52-Aprotiningen und
Restriktionskarte des rekombinanten Plasmids pNH 02.1.1

```
                      X                              A
                      h                              p
                      o                              a
                      1                              1
          ArgProAspPheCysLeuGluProProTyrThrGlyProCys -
          AATTCATCCGTCCCGACTTCTCCCTCGAGCCGCCGTACACTGGCCCCTCC
       1  -------------------------------+------------------->  50
          GTACGCAGGCCTGAAGACGGAGCTCGGCGGCATGTGACCCGGGACG


                                                  S
                                                  t
                                                  u
                                                  l
          ValAlaLeuIleIleArgTyrPheTyrAsnAlaLysAlaGlyLeuCysGln -
          GTTGCTCTCATCATCCGTTACTTCTACAATGCAAAGGCAGGCCTGTGTCA
      51  ----------+---------+----------+---------+--------->  100
          CAACGAGAGTAGTAGGCAATGAAGATGTTACGTTTCCGTCCGGACACAGT



          ThrPheValTyrGlyGlyCysArgAlaLysArgAsnAsnPheLysSerAla -
          GACCTTCGTATACGGCGGTTGCCGTGCTAAGCGTAACAACTTCAAATCCG
     101  ----------+---------+----------+---------+--------->  150
          CTGGAAGCATATGCCGCCCAACGGCACGATTCGCATTGTTGAAGTTTAGGC


          S                                   H
          s                                   1
          s                                   n
          t                                   d
          2                                   3
          GluAspCysGluArgThrCysGlyGlyAlaEnd
          CGGAAGACTGCGAACGTACTTGCGGTGGTGCTTAGTAAAGCTTG
     151  ----------+---------+----------+---------+---------
          GCCTTCTGACGCTTGCATGAACGCCACCACGAATCATTTCGAACCTAG
```

Figur 6:

DNA-Sequenz von Val-15-Leu-17-Glu-39-Glu-52-Aprotiningen
und Restriktionskarte des rekombinanten Plasmids pNH 16.1.1

```
                       X                         A
                       h                         p
                       o                         a
                       1                         1
              ArgProAspPheCysLeuGluProProTyrThrGlyProCys -
       AATTCATCCCTCCGGACTTCTGCCTCGAGCCCGCCGTACACTGGCGCCCTGC
   1   -------------------------------------------------+ 50
       GTACGCAGCCCTGAAGACGGAGCTCGGCGGCATGTGACCGCGGGACG


                                             S
                                             t
                                             u
                                             1
              ValAlaLeuIleIleArgTyrPheTyrAsnAlaLysAlaGlyLeuCysGln -
       GTTGCTCTCATCATCCGTTACTTCTACAATGCAAAGGCAGGCCTCTGTCA
  51   -------------------------------------------------+ 100
       CAACGAGAGTAGTAGGCAATGAAGATGTTACGTTTCCGTCCGGACACAGT


              ThrPheValTyrGlyGlyCysGluAlaLysArgAsnAsnPheLysSerAla -
       GACCTTCGTATACCGCGGTTGCGAAGCTAAGCGTAACAACTTCAAATCCG
 101   -------------------------------------------------+ 150
       CTGGAAGCATATGGCGCCAACGCTTCGATTCGCATTGTTGAAGTTTAGGC


       S                                     H
       x                                     i
       t                                     n
       2                                     d
              GluAspCysGluArgThrCysGlyGlyAlaEnd         3
       CGGAAGACTGCGAACGTACTTGCCGGTCGTGCTTAGTAAAGCTTG
 151   -----------------------------------------------
       GCCTTCTGACGCTTGCATGAACGGCCAGCACGAATCATTTCGAACCTAG
```

Figur 7:

DNA-Sequenz der Fragmente beta-EA10A und B,gamma-EA2A
und B,delta-EA1A und B, und delta-EA7A und B.

Sequenz des Apa1-Stu1-Fragmentes von beta-EA10A und B.
(Val-15-Leu-17-Modifikation)

```
        CysValAlaLeuIleIleArgTyrPheTyrAsnAlaLysAla
        CTGCGTTGCTCTCATCATCCGTTACTTCTACAATGCAAAGGCAGG
        ---+---------+---------+---------+---------+-----
        CCGGGACGCAACGAGAGTAGTAGGCAATGAAGATGTTACGTTTCCGTCC
```

Sequenz des Stu1-Sst2-Fragmentes gamma-EA2A und B.
(Glu-39-Modifikation)

```
      LeuCysGlnThrPheValTyrGlyGlyCysGluAlaLysArgAsnAsnPheLysSer
    -CCTGTGTCAGACCTTCGTATACGGCGGTTGCGAAGCTAAGCGTAACAACTTCAAATCCGC
      ---------+---------+---------+---------+---------+---------+
      GGACACAGTCTGGAAGCATATGCCGCCAACGCTTCGATTCGCATTGTTGAAGTTTAGG
```

Sequenz des Sst2-Bam HI-Fragmentes delta-EA1A und B.
(Met-52-Modifikation)

```
        GluAspCysMetArgThrCysGlyGlyAlaEnd
        GGAAGACTGCATGCGTACTTGCGGTGGTGCTTAGTAAAGCTTG
        -+---------+---------+---------+---------+-------
        CGCCTTCTGACGTACGCATGAACGCCACCACGAATCATTTCGAACCTAG
```

Sequenz des Sst2-Bam HI-Fragmentes delta-EA7A und B:
(Thr-52-Modifikation)

```
        GluAspCysThrArgThrCysGlyGlyAlaEnd
        GGAAGACTGCACCCGTACTTGCGGTGGTGCTTAGTAAAGCTTG
        -+---------+---------+---------+---------+-------
        CGCCTTCTGACGTGGGCATGAACGCCACCACGAATCATTTCGAACCTAG
```

Figur 8:

Konstruktion des Expressionsplasmids pES 44.1.1

1. Spaltung mit BamHI und HindIII

2. Reinigung des Vektors durch
   Gelelektrophorese

1. Abbau mit EcoRI
2. Auffüll-Reaktion (EcoRI-Stelle)
3. Ligierung mit BamHI-Linker
4. Spaltung mit BamHI und HindIII
5. Isolierung des 200 bp Fragmentes
   (Val-15-Leu-17-Glu-52-Aprotinin,
   BamHI-HindIII)

Ligierung

Figur 9:

Konstruktion des Expressionsplasmids pNH 05.1.1

pPLc24

EcoRI

BamHI
HindIII

pNH02.1.1

EcoRI

HindIII
BamHI

- Spaltung mit BamHI und HindIII

- Reinigung des Vektors durch
  Gelelektrophorese

- Spaltung mit EcoRI

- Auffüll-Reaktion (EcoRI)

- Ligierung mit BamHI-Linker

- Spaltung mit BamHI und HindIII

- Isolierung des 200 bp Fragmentes
  (Val15-Leu17-Glu52-Aprotinin)

Ligierung

pNH05.1.1

EcoRI

bamHI
EcoRI
HindIII

Figur 10:

Konstruktion des Vektors pWB 2601

Figur 11:

DNA-Sequenz des alpha-Amylase-Leaders aus Plasmid pALK1

Eco RI

```
        -100      -90       -80       -70       -60       -50
          .         .         .         .         .         .

GAATTCTCCAGTCTTCACAACAATTGAAACGAGGAAGCCTGAAGAAAGAGTAAGAGGAATT
```

```
        -40       -30       -20       -10        1        10
          .         .         .         .         .         .

TTTGACTCCGCAGTCAGTCTTCAAAAATCAAATAAGGAGTGTCAAAA ATG TTT AAA AAA
                                      SD            Met Phe Lys Lys
```

```
         20        30        40        50        60
          .         .         .         .         .

CGA TTC AAA ACC TCT TTA CTG CCG TTA TTC GCC GGA TTT TTA CTG CTG
Arg Phe Lys Thr Ser Leu Leu Pro Leu Phe Ala Gly Phe Leu Leu Leu
```

```
         70        80          90       100
          .         .    HaeIII   .         .

TTT CAT TTG GTT TTG TCA GGC CCG GCG GCT GCA AAC GCT GAA ACT GCA
Phe His Leu Val Leu Ser Gly Pro Ala Ala Ala Asn Ala Glu Thr Ala
                                               31↑
                                                 │ E.coli Prozessierungs-
                                                 │ stelle
```

```
110        120         130
  .          .   BstEII   .

CAC AAA TCG AAT GAG GTG ACC GAT
His Lys Ser Asn Glu Val Thr Asp
              41↑
                │ Bacillus-
                │ Prozessierungsstelle
```

Figur 12:

Konstruktion des Plasmids pCH 237

- Schneiden mit EcoRI und NheI
- Isolieren des Vektors

- Schneiden mit EcoRI und HaeIII
- Isolieren des 180 bp Fragmentes

- Ligieren von Fragment B

```
CCCGGCGGCTG
GGGCCGCCGACGATC
```

- Schneiden mit BamHI
- Auffüllen mit dNTP
- Ligieren des HindIII-Linkers

- Schneiden mit EcoRI und BamHI
- Isolieren des 340 bp Fragmentes

- Schneiden mit EcoRI und BamHI
- Isolieren des Vektors

Figur 13:

Konstruktion von Plasmid pCH 2472

Schneiden mit NheI&HindIII

Isolieren des Vektors

Schneiden mit XbaI und Hind III

Isolieren des 150 bp Fragmentes

(XbaI)
(NheI)

EP 0 307 592 B1

Figur 14:

Reinigung des MS2-Val-15-Leu-17-Glu-52-Aprotinin-
Fusionsproteins durch Gelfiltration auf Sephacryl$^{(R)}$ S-300
in 50 mM Tris pH 8,0, welches 6 M Harnstoff und 10 mM
beta-Mercaptoethanol enthielt. Die vereinigten Fraktionen
sind mit einem Querstrich angezeigt.

Figur 15:

Renaturierung von Val-15-Leu-17-Glu-52-Aprotinin auf CM-Sepharose$^{(R)}$. Die vereinigten Fraktionen sind mit einem Querstrich angezeigt.

Figur 16:

Reinigung von Val-15-Leu-17-Glu-52-Aprotinin auf einer Mono S$^{(R)}$ Säule. Die vereinigten Fraktionen sind mit einem Querstrich angezeigt.

Figur 17:

HPLC-Chromatographie von Val-15-Leu-17-Glu-52- und
Val-15-Leu-17-Glu-39- Glu-52-Aprotinin

Zeit (min)

3 nMol Val-15-Leu-17-Glu-52-Aprotinin wurden auf einer
Bakerbond RP-18-weitporigen Säule (250x4,6 mm) chromatographiert. Bedingungen: Fließgeschwindigkeit 0,7 ml/min;
Nachweis 210 nm; Ofentemperatur 40°C; Lösung A 0,1 % TFA,
Lösung B 0,1 % TFA/60 % ACN; Gradient: 0 min 0 % B; 10 min
0 % B, 70 min 100 % B, 80 min 0 % B; Nachweis: 210 nm.

Zeit (min)

4 nMol Val-15-Leu-17-Glu-39-Glu-52-Aprotinin wuden auf einer
Bakerbond RP-18 Säule (250x4,6 mm, weitporig) chromatographiert. Bedingungen: Fließgeschwindigkeit 0,7 ml/min; Ofentemperatur 40°C; Lösung A 0,1 % TFA, Lösung B 0,1 % TFA/60 %
ACN; Gradient: 0 min 0 % B, 10 min 0 % B, 70 min 100 % B,
80 min 0 % B; Nachweis: 210 nm.

42

Figur 18:

Chromatographie von Val-15-Leu-17-Aprotinin auf einer
Mono S$^{(R)}$ Säule.

Einzelheiten werden in Beispiel 8 beschrieben.

Fraktion Nr.

Figur 19:

Inhibierung von humaner Leukozyten-Elastase durch zunehmende Mengen an Val-15-Glu-52- und Val-15-Leu-17-Glu-52-
Aprotinin

Val-15-Glu-52-Aprotinin:                                    ------o------

Val-15-Leu-17-Glu-52-Aprotinin:                           ••••••x••••••

Konzentration von humaner Leukozyten-Elastase     $5.5 \times 10^{-9}$ M